# EUROPEAN PATENT SPECIFICATION

(11) **EP 1 477 106 B1**
(45) Date of publication and mention of the grant of the patent: **11.06.2008**
(21) Application number: 02758873.0
(22) Date of filing: 26.08.2002
(51) Int. Cl.: A61L 2/07, A61L 2/18, A61B 1/12, A61L 2/26

(54) **HIGH TEMPERATURE AND PRESSURE STEAM STERILIZATION CONTAINER FOR AN ENDOSCOPE AND ENDOSCOPE CLEANING AND STERILIZING DEVICE**
HOCHTEMPERATUR-HOCHDRUCK-DAMPFSTERILISATIONSBEHÄLTER FÜR EIN ENDOSKOP UND EINE ENDOSKOP-REINIGUNGS/STERILISIERUNSVORRICHTUNG
RECIPIENT DE STERILISATION A LA VAPEUR HAUTE PRESSION ET HAUTE TEMPERATURE POUR UNE ENDOSCOPE ET DISPOSITIF DE NETTOYAGE/STERILISATION D'UN ENDOSCOPE

(43) Date of publication of application: 17.11.2004
(73) Proprietor: Olympus Corporation, Tokyo 151-0072 (JP)
(72) Inventor: NOGUCHI, Toshiaki, Tachikawa-shi, Tokyo 190-0002 (JP); MORIYAMA, Hiroki, Akishima-shi, Tokyo 196-0032 (JP); OSHIMA, Ryu, Hachioji-shi, Tokyo 193-0832 (JP); AMANO, Shoichi, Hino-shi, Tokyo 191-0052 (JP); ISHIBIKI, Kota, Hachioji-shi, Tokyo 192-0916 (JP); MORISHITA, Koji, Mitaka-shi, Tokyo 181-0004 (JP); FUTATSUGI, Yasuyuki, Hachioji-shi, Tokyo 193-0825 (JP); HASEGAWA, Hitoshi, Kohoku-ku, Yokohama-shi, Kanagawa 222-00 (JP); NAKAGAWA, Mikihiko, Hachioji-shi, Tokyo 192-0023 (JP); YOSHIMOTO, Yosuke, Hachioji-shi, Tokyo 192-0912 (JP); TASHIRO, Yoshio, Hachioji-shi, Tokyo 192-0916 (JP); HIGUMA, Masakazu, Nirasaki-shi, Yamanashi 407-0014 (JP)
(74) Representative: von Hellfeld, Axel
(86) International application number: PCT/JP2002/008568
(87) International publication number: WO 2004/017824

(56) References cited:
- JP-A- 5 091 977
- JP-A- 7 079 907
- JP-A- 7 111 981
- JP-A- 11 009 541
- JP-A- 2000 000 292
- JP-A- 2000 300 515
- JP-A- 2001 112 857
- JP-A- 2002 045 335
- JP-A- 2002 253 477
- JP-A- 2002 253 648
- JP-U- 4 030 501
- US-A1- 2002 001 551
- US-A1- 2002 015 673
- US-A1- 2002 016 525
- US-B1- 6 354 312

## Description

### Technical Field

The present invention relates to a high temperature and pressure steam sterilization container for an endoscope, and an endoscope cleaning and sterilizing device in which autoclave sterilization (high temperature and pressure steam sterilization) is performed.

### Background Art

In general, an endoscope has been broadly used in a medical field. For the endoscope, an elongated insertion portion is inserted into a body cavity.> Accordingly, a deep part or the like in the body cavity is observed. Furthermore, when a treatment tool is used as required, a medical treatment and the like can be carried out. It is indispensable to securely disinfect and sterilize these endoscopes for medicine after use.

In recent years, autoclave sterilization (high temperature and pressure steam sterilization) has become a mainstream as means for sterilizing endoscope apparatuses. The above-described autoclave sterilization does not involve any laborious operation in a sterilization treatment. Furthermore, the treated endoscope apparatuses can be used immediately after the sterilization treatment, and additionally a running cost is low.

As the autoclave sterilization treatment, for example, an autoclave sterilization device is described in Jpn. Pat. Appln. KOKAI Publication No. JP 5-285103. Here, the device is described in which the endoscope capable of performing high temperature and pressure steam sterilization is projected to perform the autoclave sterilization (high temperature and pressure steam sterilization).

As a typical environmental condition of the autoclave sterilization treatment, there is U.S. standard ANSI/AAMI ST37-1992 authorized by U.S. Standards Association and issued by Medical Devices Development Association. The environmental condition standardized here include a sterilization process at 132°C for four minutes for a pre-vacuum type or a sterilization process at 132°C for ten minutes for a gravity type.

The above-described environmental condition of the autoclave sterilization is very severe for an electronic endoscope which is a precision electronic apparatus including image pickup devices such as CCD. To realize the electronic endoscope which has resistance to the condition and which is capable of performing the autoclave sterilization (high temperature and pressure steam sterilization), unlike an endoscope usable only in another disinfecting and sterilizing means, various measures have to be taken such as a high-pressure measure, a high-temperature measure, and a steam measure.

Especially, since the endoscope insertion portion is to be inserted into a patient's body, various subtle characteristics such as flexibility and snapping property are required. An elongated flexible tubular portion is disposed behind a tip hard portion disposed in a tip of the endoscope insertion portion. Moreover, the flexible tubular portion tends to be easily affected by high pressure, high temperature, or steam as compared with the tip hard portion, and a further advanced measure is required.

Moreover, during the autoclave sterilization treatment, not only the endoscope but also the other peripherals of the endoscope are disposed together in a tray and projected into an autoclave sterilization device. Here, examples of the peripherals of the endoscope to be projected into the autoclave sterilization device include various buttons for feeding air or water and the like, detached from the endoscope, a water proof cap to be attached to the endoscope, and treatment tools such as forceps.

Furthermore, to perform the autoclave sterilization treatment in a state in which the above-described peripherals of the endoscope including the treatment tools such as the forceps are superposed upon soft portions such as the endoscope insertion portion and a universal cord or they contact one another, there is also a possibility that the soft endoscope insertion portion is contacted and pressed by the treatment tools.

Therefore, the measure against the high pressure, high temperature, or steam is required to be taken in the constitution of the endoscope insertion portion itself as described above at the time of the autoclave sterilization treatment. Furthermore, in addition to this measure, it is also important to devise a projection mode of an object to be sterilized/treated during the projection into the autoclave sterilization device, so that the endoscope insertion portion is prevented from being damaged by the peripherals of the endoscope.

However, a capacity of a sterilization treatment chamber is comparatively small in the conventional autoclave sterilization device having such a size that the device can be disposed in an endoscopic inspection chamber. Therefore, when the endoscope including the elongated soft insertion portion is projected into the sterilization treatment chamber, the insertion portion needs to be bundled in a round shape and projected.

In this manner, in the above-described conventional autoclave sterilization device, when the insertion portion of the endoscope is projected in an excessively small round state, the soft insertion portion is probably damaged. For example, there is also a possibility that an influence of bend deformation by heat is exerted. Therefore, it is important to store the endoscope in a predetermined shape in the tray or a container.

In general, the endoscope needs to be cleaned before the autoclave sterilization treatment. At this time, to clean the endoscope in an automatic cleaner, the endoscope has to be stored in a predetermined mode also in the cleaner.

That is, a user stores and cleans the endoscope in the predetermined mode in the cleaner. Thereafter, the endoscope is taken out of the cleaner, and set in the tray (or container) for the autoclave sterilization treatment. Also at this time, an operation for storing the endoscope in the predetermined mode in the tray (or container) for the autoclave sterilization treatment needs to be performed, and therefore the operation of the autoclave sterilization treatment becomes laborious.

### Disclosure of Invention

The present invention has been developed in consideration of these circumstances, and an object thereof is to provide a high temperature and pressure steam sterilization container for an endoscope, and an endoscope cleaning and sterilizing device in which an operation is facilitated in a case where a high temperature and pressure steam sterilization treatment is performed after cleaning the endoscope used in an endoscopic inspection.

Moreover, another object of the present invention is to provide an endoscope cleaning and sterilizing device whose entire cost is reduced and which is entirely miniaturized so that the endoscope cleaning and sterilizing device can be disposed even in a general consultation room where diagnosis by the endoscope is conducted and which is capable of reducing a time required for operations of cleaning, disinfecting, and sterilizing the endoscope or the like, performed between cases of endoscopic diagnosis and which is capable of enhancing efficiency of the endoscopic diagnosis.

Furthermore, another object is to provide an endoscope cleaning and sterilizing device capable of easily maintaining a sterilized state of an endoscope subjected to the respective cleaning, disinfecting, and sterilizing operations.

The document JP 2002 045335 A discloses an endoscope cleaning and sterilizing device comprising a container, a cleaning section and a high temperature and pressure steam sterilization section.

The objects above are obtained with a device according to the appended claims.

### Brief Description of Drawings

FIG. 1 is a schematic constitution diagram of a whole endoscope device including a high temperature and pressure steam sterilization container for an endoscope of a first embodiment of the present invention;
FIG. 2 is an explanatory view showing an outline of an endoscope cleaning and sterilizing system of the present embodiment;
FIG. 3A is a plan view showing a tray in which an endoscope is stored;
FIG. 3B is a vertical sectional view showing an inclined section of a floor surface in the vicinity of an operation section in an endoscope storage portion of the tray;
FIG. 4 is a perspective view showing an appearance of a sterilization container according to a second embodiment of the present invention;
FIG. 5 is a plan view showing an opened state of a lid of the sterilization container in which the endoscope is stored;
FIG. 6 is a front view showing a state in which the endoscope is hung and supported from a hanger device of a trolley;
FIG. 7 is a perspective view showing a schematic constitution of a whole cleaning, disinfecting and sterilizing device of a third embodiment of the present invention;
FIG. 8A is a perspective view showing a tray main body of a structure type in which a tray lower unit is separated from a tray lid in the cleaning, disinfecting and sterilizing device of the third embodiment;
FIG. 8B is a perspective view showing the tray main body of a structure type in which the tray main body is integrated with the lid;
FIG. 9 is a vertical sectional view showing a schematic constitution inside the cleaning, disinfecting and sterilizing device of the third embodiment;
FIG. 10A is a vertical sectional view showing an inner constitution of the tray main body in the cleaning, disinfecting and sterilizing device of the third embodiment;
FIG. 10B is a side view showing one side surface of the tray main body;
FIG. 10C is a side view showing the other side surface of the tray main body;
FIG. 11 is a vertical sectional view of a main part showing an open state of a valve for opening and closing the pipe line of the tray main body in the cleaning, disinfecting and sterilizing device of the third embodiment;
FIG. 12 is a vertical sectional view of the main part showing a closed state of the valve for opening and closing the pipe line of the tray main body in the cleaning, disinfecting and sterilizing device of the third embodiment;
FIG. 13 is a schematic constitution diagram showing an electric circuit of a tray side transmission and reception unit and a main body side transmission and reception unit in the cleaning, disinfecting and sterilizing device of the third embodiment;
FIG. 14A is a vertical sectional view of the main part showing a state in which the tray main body in the cleaning, disinfecting and sterilizing device of the third embodiment is conveyed to a sterilization unit section;
FIG. 14B is a vertical sectional view of the main part showing an operation state of a motor for lifting up and down;
FIG. 14C is a vertical sectional view of the main part showing a state in which the tray main body is conveyed into a chamber for sterilization;
FIG. 15 is a vertical sectional view of the main part showing the open state of the valve for opening and closing the pipe line in the cleaning, disinfecting and sterilizing device of a fourth embodiment of the present invention;
FIG. 16 is a vertical sectional view of the main part showing the closed state of the valve for opening and closing the pipe line in the cleaning, disinfecting and sterilizing device of the fourth embodiment;
FIG. 17A is a vertical sectional view of the main part showing a state in which the tray main body in the cleaning, disinfecting and sterilizing device of a fifth embodiment of the present invention is conveyed to the sterilization unit section;
FIG. 17B is a vertical sectional view of the main part showing an operation state of a float for lifting up and down;
FIG. 17C is a vertical sectional view of the main part showing a state in which the tray main body is conveyed into the chamber for sterilization;
FIG. 18 is a perspective view showing the cleaning, disinfecting and sterilizing device of a sixth embodiment of the present invention;
FIG. 19 is a vertical sectional view of the main part showing the closed state of a check valve of the tray main body in the cleaning, disinfecting and sterilizing device of a seventh embodiment of the present invention;
FIG. 20A is a plan view showing a connector on a device side in the cleaning, disinfecting and sterilizing device of the seventh embodiment;
FIG. 20B is a vertical sectional view of the connector on the device side;
FIG. 20C is a vertical sectional view of the main part showing the open state of the check valve of the tray main body;
FIG. 21 is a vertical sectional view of the main part showing a modification of the check valve of the tray main body in the cleaning, disinfecting and sterilizing device of the seventh embodiment;
FIG. 22A is a side view showing the tray main body on a belt conveyor in the cleaning, disinfecting and sterilizing device of an eighth embodiment of the present invention;
FIG. 22B is a vertical sectional view showing a bag-shaped film with which the tray main body is covered;
FIG. 22C is a vertical sectional view showing a sealed of the tray main body covered with the bag-shaped film;
FIG. 23A is a side view of the tray main body on the belt conveyor showing a modification of the cleaning, disinfecting and sterilizing device of the eighth embodiment;
FIG. 23B is a vertical sectional view showing a sheet film with which an opening of the tray main body is covered; and
FIG. 23C is a vertical sectional view showing the sealed state of the tray main body covered with the sheet film.

### Best Mode for Carrying Out the Invention

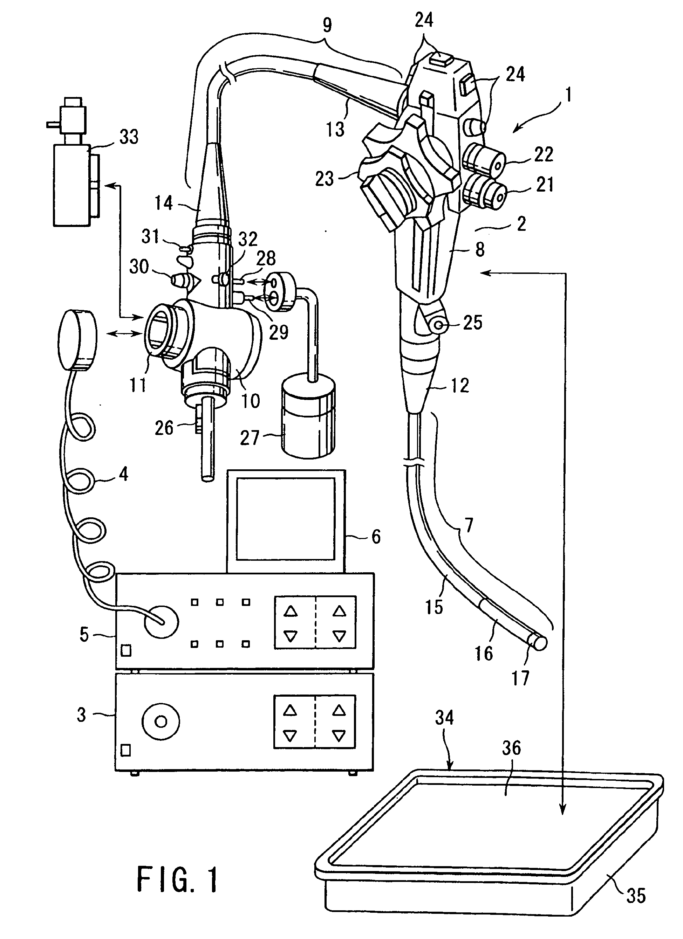

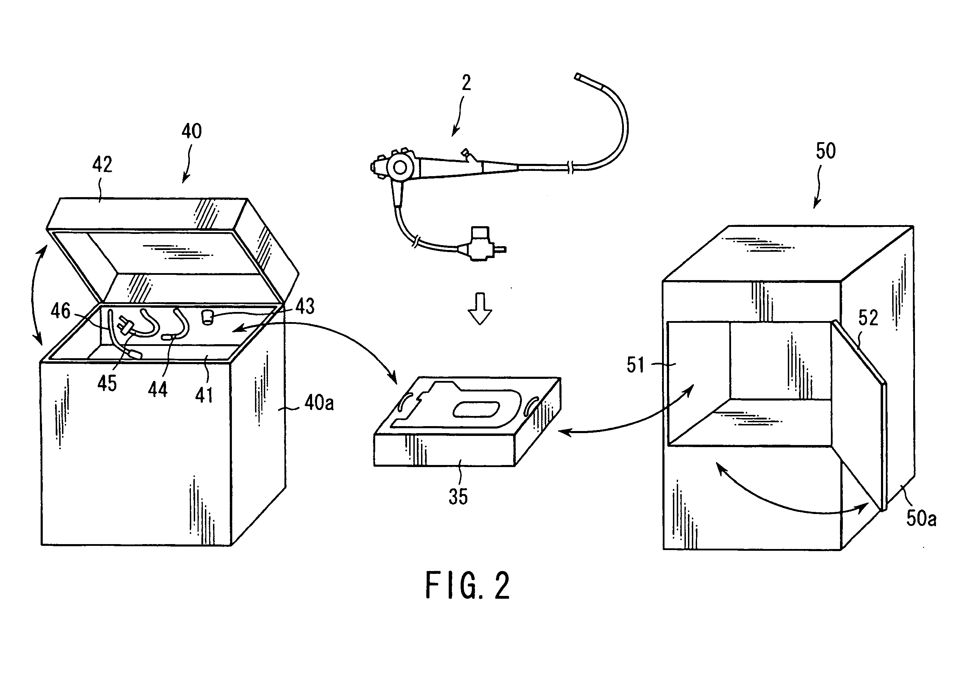

A first embodiment of the present invention will be described hereinafter with reference to FIGS. 1 to 3B. FIG. 1 is a schematic constitution diagram of a whole endoscope device including a first embodiment of the present invention, and FIG. 2 is an outline view of an endoscope cleaning and sterilizing system of the present embodiment.

As shown in FIG. 1, an endoscope device 1 including the first embodiment of the present invention is mainly constituted of an endoscope 2, a light source device 3, a video processor 5, and a monitor 6. The endoscope 2 includes image pickup means (not shown). The light source device 3 is detachably attached to the endoscope 2 to supply an illuminative light to a light guide disposed in the endoscope 2. The video processor 5 is connected to the endoscope 2 via a signal cable 4 to control the image pickup means of the endoscope 2, and further processes a signal obtained from the image pickup means to output a standard video signal. The monitor 6 inputs the video signal from the video processor 5 to display an endoscope image.

It is to be noted that the endoscope 2 is constituted to be cleaned after used in observation or treatment, and can further be sterilized in autoclave sterilization (high temperature and pressure steam sterilization) after cleaned.

The endoscope 2 is provided with an elongated insertion portion 7 having flexibility. An operation portion 8 is disposed on a base-end side of the insertion portion 7. Furthermore, a side portion of the operation portion 8 is connected to one end of a universal cord 9 having flexibility. A connector portion 10 is disposed on the other end of the universal cord 9. This connector portion 10 is detachably connectable to the light source device 3.

An electric connector portion 11 is disposed on the side portion of this connector portion 10. This electric connector portion 11 is detachably connectable to one end of the signal cable 4. The other end of the signal cable 4 is connectable to the video processor 5.

The electric connector portion 11 is provided with a ventilation portion (not shown) which connects the inside of the endoscope 2 to the outside.

An insertion portion side bend preventive member 12 is disposed in a connecting portion between the insertion portion 7 and the operation portion 8. This bend preventive member 12 includes an elastic member which prevents a rapid bend of the connecting portion between the insertion portion 7 and the operation portion 8. Similarly, an operation portion side bend preventive member 13 is disposed in the connecting portion between the operation portion 8 and the universal cord 9. Furthermore, a connector portion side bend preventive member 14 is disposed in the connecting portion between the universal cord 9 and the connector portion 10.

The insertion portion 7 is constituted of an elongated flexible tubular portion 15, a curved portion 16, and a tip portion 17. The flexible tubular portion 15 is a soft portion having the flexibility. The curved portion 16 is disposed on the tip side of the flexible tubular portion 15. The curved portion 16 can be curved by a remote operation from the operation portion 8.

The tip portion 17 is disposed on the tip of the curved portion 16, and is provided with an optical observation system, an optical illuminating system, a solution feeding port and air or water feeding nozzle, a suction port and the like.

Furthermore, a pipe line for air or water feed, or a treatment tool passing channel which also serves as a suction pipe line and the like are disposed in the insertion portion 7. Here, the tip of the pipe line for air or water feed is connected to a solution feeding port and air or water feeding nozzle of the tip portion 17. The tips of the suction pipe line and treatment tool passing channel are connected to the suction port.

The operation portion 8 is provided with an air or water feed operation button 21, a suction operation button 22, two curving operation knob 23, a plurality of remote switches 24, and a treatment tool insertion port 25. The air or water feed operation button 21 switches and operates the air or water feed in the pipe line for air or water feed in the insertion portion 7. The suction operation button 22 switches and operates a suction operation from a suction pipe line. Two curving operation knobs 23 are coaxially disposed in two upper and lower stages. Moreover, one curving operation knob 23 curves and operates the curved portion 16, for example, in a vertical direction. The other curving operation knob 23 curves and operates the curved portion 16, for example, in a right or left direction. The plurality of remote switches 24 remotely operate the video processor 5. The treatment tool insertion port 25 is an opening communicating with the base end of the treatment tool passing channel.

Moreover, a cleaning solution or gas is spouted toward an observation window of the optical observation system (not shown) from the solution feeding port and air or water feeding nozzle (not shown) of the tip portion 17 by the switching operation of the air or water feeding by the air or water feeding operation button 21. A solution in a body cavity is sucked from the suction port (not shown) of the tip portion 17 by the operation of the suction operation button 22. Furthermore, a treatment tool is passed through the treatment tool passing channel (not shown) disposed in the insertion portion 7 from the treatment tool insertion port 25 of the operation portion 8. Moreover, this treatment tool is extended to the outside of the channel from the suction port (not shown) of the tip portion 17 which is the opening on the tip side of the treatment tool passing channel.

The connector portion 10 is further provided with a gas supply cap 26, a water feeding tank pressurizing cap 28 and solution supply cap 29, a suction cap 30, and an injection cap 31. The gas supply cap 26 is detachably connected to a gas supply source (not shown) built in the light source device 3. The water feed tank pressurizing cap 28 and solution supply cap 29 are detachably connected to a water feed tank 27 which is a solution supply source. The suction cap 30 is connected to a suction source (not shown). Moreover, a suction force from the suction source is exerted onto the suction port of the tip portion 17 via the suction cap 30 and suction pipe line to perform the suction via the suction port of the tip portion 17. The injection cap 31 is connected to water feeding means (not shown). Moreover, water is fed via the solution feeding port of the tip portion 17 via the injection cap 31 and the pipe line for air or water feeding from the water feeding means.

Moreover, the connector portion 10 is further provided with an earth terminal cap 32. The earth terminal cap 32 returns a leak current to a high frequency treatment device in a case where a high frequency leak current is generated in the endoscope during a high frequency treatment.

Furthermore, a regulating portion (not shown) is formed in accordance with the shape of the endoscope 2 in the connector portion 10. The regulating portion is formed in such a manner that the respective portions of the endoscope 2 are stored in predetermined positions. Moreover, the regulating portion is provided with an insertion portion regulating portion (not shown) in which the insertion portion 7 is stored.

The electric connector portion 11 is detachably connectable to a waterproof cap provided with a pressure adjustment valve (hereinafter referred to as the waterproof cap) 33. This waterproof cap 33 is provided with the pressure adjustment valve (not shown).

Moreover, during the autoclave sterilization (high temperature and pressure steam sterilization) treatment of the endoscope 2, a container case for sterilization 34 which stores the endoscope 2 is used.

The container case 34 is constituted of a tray 35 which is a high temperature and pressure steam sterilization container for the endoscope, and a back lid member 36. A containing concave portion which stores the endoscope insertion portion as described later is formed in the tray 35. A plurality of ventilation ports (not shown) are disposed in these tray 35 and back lid member 36. Moreover, vapor is capable of passing in the container case 34 through these ventilation ports at the time of the autoclave sterilization (high temperature and pressure steam sterilization).

As described above, in U.S. standards ANSI/AAMI ST37-1992 which is a typical treatment condition of the high temperature and pressure steam sterilization, a sterilization process at 132°C for four minutes for a pre-vacuum type, and a sterilization process at 132°C for ten minutes for a gravity type are set.

A temperature condition of the sterilization process of the autoclave sterilization (high temperature and pressure steam sterilization) differs with a type of an autoclave sterilization device (not shown) or a time of the sterilization process. Moreover, in general, the temperature condition is set in a range of about 115°C to 138°C. The condition can be set to about 142°C in some of the sterilization devices. A time condition differs with the temperature condition of the sterilization process. In general, the condition is set to about 3 to 60 minutes. The condition may also be set to about 100 minutes depending on the type of the sterilization device. The pressure in a sterilization chamber in this process is set to about +0.2 MPa with respect to an atmospheric pressure.

The autoclave sterilization (high temperature and pressure steam sterilization) process of the general pre-vacuum type includes a pre-vacuum process and a sterilization process. In the pre-vacuum process, the inside of the sterilization chamber in which a sterilization object apparatus is stored is brought into a reduced pressure state before the sterilization process. In the sterilization process, a high pressure and temperature steam is fed into the sterilization chamber to perform the sterilization after the pre-vacuum process.

In the former pre-vacuum process, steam is permeated into details of the sterilization object apparatus at the time of the latter sterilization process. Here, the pressure inside the sterilization chamber is reduced so that the high pressure and temperature steam spreads entirely in the sterilization object apparatus. The pressure in the sterilization chamber in the pre-vacuum process is generally set to about -0.07 MPa to 0.09 MPa with respect to an atmospheric pressure.

A drying process is sometimes included after the sterilization process. In this drying process, the inside of the sterilization chamber is brought into the reduced pressure state again after the sterilization process. Accordingly, the sterilization object apparatus after the sterilization is dried.

In this drying process, the pressure inside the sterilization chamber is reduced to discharge steam from the sterilization chamber, and the drying of the sterilization object apparatus in the sterilization chamber is promoted. The pressure in the sterilization chamber in this process is generally set to about -0.07 MPa to 0.09 MPa with respect to the atmospheric pressure.

During the autoclave sterilization (high temperature and pressure steam sterilization) of the endoscope 2, the waterproof cap 33 is attached to the electric connector portion 11. At this time, the pressure adjustment valve (not shown) of the waterproof cap 33 is closed. Moreover, in this state, the ventilation port of the electric connector portion 11 is blocked by the waterproof cap 33, and the inside of the endoscope 2 is sealed and closed from the outside in a watertight manner.

In a sterilization method including the pre-vacuum process, in this pre-vacuum process the pressure in the sterilization chamber decreases and a pressure difference is made such that the pressure of the outside of the endoscope 2 is lower than that of the inside. The pressure adjustment valve of the waterproof cap 33 opens by the pressure difference at this time. Therefore, the inside of the endoscope 2 communicates with the outside via the ventilation port of the electric connector portion 11. Accordingly, a large difference is prevented from being made between the pressure inside the endoscope 2 and that in the sterilization chamber. As a result, the endoscope 2 does not break by the pressure difference between the inside and the outside.

In the sterilization process, the sterilization chamber is pressurized. At this time, when the pressure difference is made such that the outside of the endoscope 2 has a pressure higher than that of the inside, the pressure adjustment valve of the waterproof cap 33 closes. Accordingly, the high pressure and temperature steam does not positively enter the endoscope 2 via the waterproof cap 33 and the ventilation port of the electric connector portion 11.

However, an outer shell of the flexible tubular portion 15 is formed of a macromolecular material. The connecting portion of an exterior member of the endoscope 2 is provided with an O ring and the like formed of a fluorine or silicon rubber which is sealing means. Moreover, the high temperature and pressure steam gradually enters the endoscope 2 from the outer shell of the flexible tubular portion 15 or the O ring or the like of the connecting portion of the exterior member of the endoscope 2.

At this time, a pressure reduced in the pre-vacuum process and a pressure added in the sterilization process act on the exterior member of the endoscope 2 in an added state. Therefore, a pressure directed to the inside from the outside is generated in the exterior member of the endoscope 2.

In a method including a pressure reducing process after the sterilization process, in the pressure reducing process, the pressure in the sterilization chamber decreases and the pressure difference is made such that the pressure of the outside of the endoscope 2 is lower than that of the inside. At this time, the pressure adjustment valve of the waterproof cap 33 opens substantially simultaneously with the generation of the pressure difference. Therefore, the inside of the endoscope 2 communicates with the outside via the ventilation port of the electric connector portion 11 to prevent the large difference from being made between the pressure inside the endoscope 2 and that in the sterilization chamber. Accordingly, the endoscope 2 does not break by the pressure difference between the inside and the outside.

Furthermore, the pressure reducing process ends, the inside of the sterilization chamber is pressurized to make the pressure difference such that the pressure inside the endoscope 2 is higher than that of the outside, and the pressure adjustment valve then closes.

As described above, when all the processes of the high temperature and pressure steam sterilization end, the pressure directed to the inside from the outside is generated in the exterior member of the endoscope 2 because of the reduced pressure in the pressure reducing process.

Moreover, when the waterproof cap 33 is removed from the electric connector portion 11, the inside of the endoscope 2 communicates with the outside via the ventilation port of the electric connector portion 11 to bring the inside of the endoscope 2 into the atmospheric pressure. Accordingly, a load by the pressure generated in the exterior member of the endoscope 2 is eliminated.

In the present embodiment, the endoscope 2 is stored in the tray 35 which is a container for performing the autoclave sterilization (high temperature and pressure steam sterilization) with respect to the above-described endoscope 2, and is stored in an endoscope cleaner described later in this state, so that the endoscope 2 can be cleaned.

Moreover, as shown in FIG. 2, the endoscope cleaning and sterilizing system of the present embodiment is provided with an endoscope cleaner 40 for cleaning the endoscope 2, and an autoclave device 50 for performing the autoclave sterilization (high temperature and pressure steam sterilization) of the endoscope 2. The endoscope cleaner 40 is provided with a cleaning bath 41 whose upper surface is opened in an upper part of a cleaner main body 40a. A waterproof lid 42 is openably and closably disposed facing the cleaning bath 41 in the upper surface opening of the cleaning bath 41. Moreover, the waterproof lid 42 is closed in a state in which the tray 35 including the endoscope 2 stored therein is stored in the cleaning bath 41, and accordingly the sealing is possible.

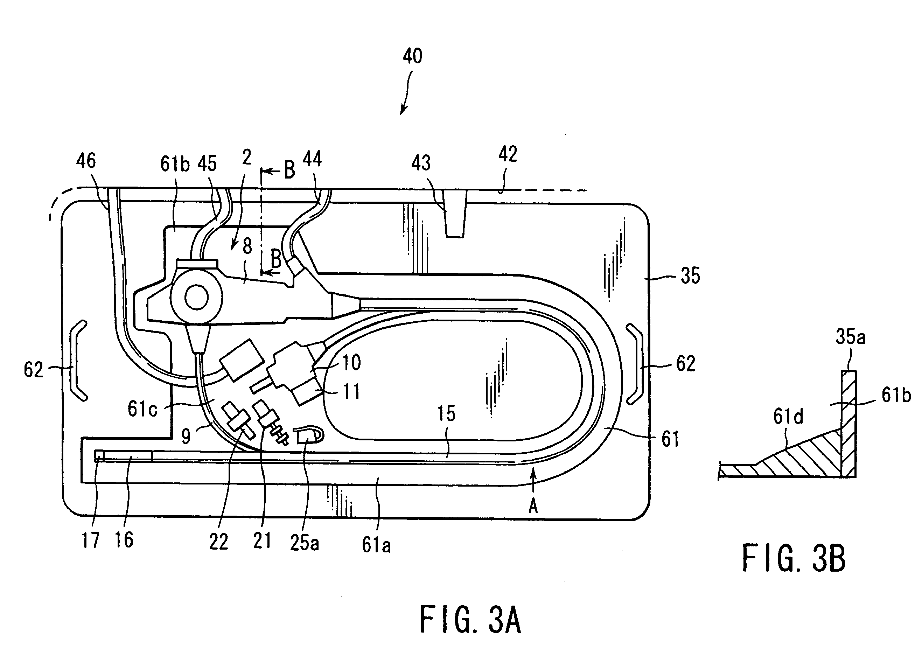

Moreover, a water injection port 43, a treatment channel cleaning tool 44, an air or water feeding cleaning tool 45, and a water discharge tool 46 are disposed on the side wall inside the cleaning bath 41. The water injection port 43 guides liquids such as the cleaning solution and water into the cleaning bath 41. As shown in FIG. 3A, the treatment channel cleaning tool 44 is detachably attached to the treatment tool insertion port 25 of the endoscope 2. Moreover, the inside of the treatment tool passing channel of the endoscope 2 is cleaned by the treatment channel cleaning tool 44. The air or water feeding cleaning tool 45 is detachably attached to an air or water feeding cylinder after the air or water feeding operation button 21 and suction operation button 22 of the endoscope 2 are removed. Moreover, the inside of the air or water feeding pipe line of the endoscope 2 is cleaned by the air or water feeding cleaning tool 45. The water discharge tool 46 discharges the liquids such as the cleaning solution and water inside the cleaning bath 41.

It is to be noted that a cleaning tool for constituting further pipe lines and the like other than these lines may also be disposed in the side wall inside the cleaning bath 41 in accordance with a function of the endoscope.

The tray 35 is stored in the cleaning bath 41 of the endoscope cleaner 40 in a state in which the endoscope 2 is stored. In this state, the endoscope cleaner 40 is capable of cleaning the endoscope 2 in the tray 35.

Moreover, the autoclave device 50 is provided with a chamber 51 in which the front surface of an autoclave device main body 50a is opened. An openable closing door 52 is disposed in the front surface opening of the chamber 51. Moreover, the tray 35 in which the endoscope 2 is stored is stored in the chamber 51 and the opening and closing door 52 is sealed and closed in an airtight manner. The autoclave sterilization (high temperature and pressure steam sterilization) is performed in this state.

It is to be noted that the autoclave device 50 is further provided with a water storage tank (not shown), a steam generation device, a suction pump, a plurality of switch valves, a control device and the like. In the water storage tank, water for the high temperature and pressure steam is stored. The steam generation device generates the high temperature and pressure steam with the water of this water storage tank. The suction pump exhausts the steam in the chamber 51 or another gas in the pressure reducing process and the like. The plurality of switch valves switch these operations. The control device controls the flow of the steam or another fluid.

The tray 35 in which the endoscope 2 is stored is stored in the chamber 51 of the autoclave device 50, and the autoclave sterilization (high temperature and pressure steam sterilization) of the endoscope 2 can be performed in the tray 35. It is to be noted that the tray 35 is formed of a material having resistance to the high temperature and pressure steam at the autoclave sterilization (high temperature and pressure steam sterilization) time.

FIG. 3A shows a state in which the endoscope 2 is stored in the tray 35 and the tray 35 is stored in the cleaning bath 41 of the endoscope cleaner 40. As shown in FIG. 3A, an endoscope storage portion 61 which is a containing concave portion for storing the endoscope 2 is formed in the tray 35. The bottom and side surfaces of the endoscope storage portion 61 are substantially smooth surfaces. When the endoscope 2 is stored in the endoscope storage portion 61 with some clearance, the stored endoscope operation portion 8 is not slid largely, and storage mode (bent shape) of the universal cord 9 or the flexible tubular portion 15 is automatically determined.

It is to be noted that a depth of the endoscope storage portion 61 in the tray 35 is set to be sufficiently larger than a height (width) of the flexible tubular portion 15, universal cord 9, operation portion 8, and connector portion 10 of the endoscope 2. Accordingly, when the flexible tubular portion 15, universal cord 9, operation portion 8, and connector portion 10 of the endoscope 2 are stored in the endoscope storage portion 61 of the tray 35, each portion of the endoscope 2 is sufficiently lower than an upper surface position of a peripheral wall portion 35a of the endoscope storage portion 61.

Moreover, the flexible tubular portion 15 of the endoscope insertion portion 7 is rounded and disposed in the endoscope storage portion 61. This is because the endoscope 2 has varieties of short and long flexible tubular portions 15. Moreover, to dispose the endoscope 2 in the endoscope storage portion 61, a device type including the long flexible tubular portion 15 cannot be stored in the comparatively small-sized autoclave sterilization device 50.

Furthermore, the flexible tubular portion 15 of the insertion portion 7 is formed of a soft resin. Therefore, when the autoclave sterilization (high temperature and pressure steam sterilization) is performed in the autoclave device 50 in a state in which the flexible tubular portion 15 of the insertion portion 7 is rounded and stored and disposed in the endoscope storage portion 61 of the tray 35, there is a possibility that the flexible tubular portion 15 has some curl. Therefore, a substantially straight flexible tubular portion storage concave portion 61a is formed in the endoscope storage portion 61. A tip side portion of the flexible tubular portion 15, that is, the tip portion 17 of the endoscope insertion portion 7 from a portion shown by an arrow A in FIG. 3A is disposed in a substantially straight form in the flexible tubular portion storage concave portion 61a. Accordingly, even when the flexible tubular portion 15 of the endoscope insertion portion 7 obtains some curl by the autoclave sterilization treatment, as little trouble as possible is caused in an insertion property of the endoscope insertion portion 7 in an inspection.

It is to be noted that the endoscope insertion portion 7 has a length, for example, of about 133 cm to 168 cm in the endoscope for a lower digestive duct. In general, when the endoscope insertion portion 7 is inserted into a cecum portion from anus of large intestine, and when an excessive deflection of the endoscope insertion portion 7 is removed as much as possible, it is said that the endoscope insertion portion 7 is inserted by about 70 cm from the tip portion 17. Moreover, the portion of about 70 cm from the tip portion 17 is an important portion to be inserted into almost all patients.

Therefore, in the present embodiment, when the portion on the tip side of the flexible tubular portion 15, that is, the portion of about 70 cm from the tip portion 17 is stored in the substantially straight form in the flexible tubular portion storage concave portion 61a of the endoscope storage portion 61, the portion of the flexible tubular portion 15 on the tip side is prevented from being curled.

Moreover, a small matter containing concave portion 61c is formed in the vicinity of the storage of the connector portion 10 of the endoscope 2 in the endoscope storage portion 61 of the present embodiment. In the small matter containing concave portion 61c, small matters such as the air or water feeding operation button 21 detached from the endoscope 2, the suction operation button 22, and a treatment tool plug 25a to be attached to the treatment tool insertion port 25 at an inspection time are stored.

It is to be noted that these small matters do not have to be necessarily stored and disposed in the endoscope storage portion 61 together with the endoscope 2. For example, when the autoclave sterilization (high temperature and pressure steam sterilization) and the cleaning are possible, the small matter containing portion may also be disposed in a place separate from the endoscope storage portion 61 in the tray 35. Furthermore, the treatment tools such as forceps are stored in the tray 35, and may also be subjected to the autoclave sterilization (high temperature and pressure steam sterilization) and cleaned together with the endoscope 2.

Moreover, as shown in FIG. 3B, an inclined portion 61d is formed by inclining a floor surface of the endoscope storage portion 61 in a slant shape along the peripheral wall portion 35a with respect to a horizontal surface in the vicinity of an operation portion containing concave portion 61b in which the operation portion 8 of the endoscope 2 is stored in the endoscope storage portion 61. Furthermore, when the operation portion 8 of the endoscope 2 is stored in the operation portion containing concave portion 61b of the endoscope storage portion 61, the operation portion 8 is disposed in a tilted state by the inclined portion 61d. Accordingly, the operation portion is stored and disposed in such a manner that the opening of the treatment tool insertion port 25 of the endoscope operation portion 8 and the opening of the air or water feeding cylinder after removing the air or water feeding operation button 21 and suction operation button 22 are directed obliquely upwards from a horizontal direction. At this time, the operation portion is stored and disposed in such a manner that the treatment tool insertion port 25 opening of the operation portion 8 or the air or water feeding cylinder opening is directed in a gravity direction (vertical downward direction) or obliquely upwards from the horizontal direction with respect to the gravity direction (vertical downward direction).

It is to be noted that the directions of opposite ends of each pipe line of the endoscope 2 to be stored in the autoclave device 50 have not heretofore been considered. Here, for example, supposing that the autoclave sterilization is performed with respect to the endoscope 2 that can be autoclave-sterilized without the pre-vacuum process, and when all the openings of the respective pipe lines of the endoscope 2 are directed in the gravity direction, there is possibility that the steam does not sufficiently spread in the pipe lines.

On the other hand, in the present embodiment, the treatment tool insertion port 25 opening of the operation portion 8 or the air or water feeding cylinder opening is directed in the gravity direction (vertical downward direction) or obliquely upwards from the horizontal direction with respect to the gravity direction (vertical downward direction) by the inclined portion 61d of the operation portion containing concave portion 61b. Therefore, the steam easily enters the pipe line of the endoscope 2 and sufficiently spreads by the gravity. The opening of the other end of the channel is disposed, for example, in the tip portion 17. Similarly in this case, the inclined portion is disposed in the floor surface of the storage concave portion, and accordingly for the similar reason the operation portion is stored and disposed in such a manner that the opening is directed in the gravity direction (vertical downward direction) or obliquely upwards from the horizontal direction with respect to the gravity direction (vertical downward direction). Accordingly, in the present embodiment, the steam sufficiently spreads in the pipe line in the endoscope 2 even in the autoclave sterilization without pre-vacuum process.

Moreover, handles 62 are disposed on the opposite ends of the upper surface of the tray 35. When these handles 62 are grasped, the tray 35 is easily transported.

Furthermore, when the tray 35 is stored in the cleaning bath 41, the treatment tool insertion port 25 of the endoscope 2 is connected to the treatment channel cleaning tool discharge tool 46, and the air or water feeding cylinder after removing the air or water feeding operation button 21 and suction operation button 22 is connected to the air or water feeding cleaning tool 46. The tip of the discharge tool 46 is laid on the bottom surface of the concave portion of the endoscope storage portion 61.

Next, a function of the endoscope cleaning and sterilizing system constituted as described above will be described.

The user detaches the air or water feeding operation button 21 and suction operation button 22 from the endoscope 2 after ending endoscopic inspection. Moreover, the endoscope 2 and the members and the like detached from the endoscope 2 are stored in predetermined places of the endoscope storage portion 61 of the tray 35. At this time, the small matters such as the air or water feeding operation button 21, suction operation button 22, and treatment tool plug 25a to be attached to the treatment tool insertion port 25 at the inspection time are stored in the small matter containing concave portion 61c.

Thereafter, the tray 35 is stored in the cleaning bath 41 of the endoscope cleaner 40. Moreover, the user connects the air or water feeding cylinder from which the air or water feeding operation button 21 and suction operation button 22 of the endoscope 2 have been removed to the air or water feeding cleaning tool 45. Furthermore, the treatment channel cleaning tool 44 is attached to the treatment tool insertion port 25 of the endoscope 2, and the water discharge tool 46 is disposed in the predetermined place of the endoscope storage portion 61 of the cleaning bath 41.

After ending preparation for the cleaning of the endoscope in this manner, the user closes the waterproof lid 42, and operates the endoscope cleaner 40 to clean the endoscope. After the end of the cleaning, the user removes the tray 35 from the endoscope cleaner 40. Thereafter, the tray 35 may also be covered with the lid member 36, or a peel pack.

Subsequently, the user stores the tray 35 in which the endoscope 2 and the members and the like detached from the endoscope 2 remain to be stored in the chamber 51 in the autoclave device 50 to perform the autoclave sterilization (high temperature and pressure steam sterilization).

At this autoclave sterilization time, while the flexible tubular portion 15 of the endoscope insertion portion 2 is bent in a predetermined shape and stored in the endoscope storage portion 61 of the tray 35, the autoclave sterilization is performed. At this time, the portion of the flexible tubular portion 15 on the tip side, that is, the tip portion 17 of the endoscope insertion portion 7 from the portion shown by the arrow A in FIG. 3A is disposed in the substantially straight form in the substantially straight flexible tubular portion storage concave portion 61a of the endoscope storage portion 61. Therefore, even if a bent predetermined portion of the flexible tubular portion 15 has some curl, a main portion largely influencing the inserting property of the flexible tubular portion 15 on the tip side is kept to be substantially straight, and therefore the inserting property of the endoscope insertion portion 7 is not adversely affected.

Moreover, for the autoclave device 50, for a device which does not include the pre-vacuum process as the case may be, or in a case where the pre-vacuum process can be performed but the user sets the device not to perform the pre-vacuum process, the chamber 51 is filled with the steam by the gravity.

At this time, as described above, since the treatment tool insertion port 25 opening of the endoscope operation portion 8 or the air or water feeding cylinder opening is directed in the gravity direction (vertical downward direction) or obliquely upwards from the horizontal direction with respect to the gravity direction (vertical downward direction), the steam easily enters the pipe line of the endoscope 2 by the gravity. Since the opening of the other end disposed in the tip portion 17 of the channel is directed in the gravity direction (vertical downward direction) or obliquely upwards from the horizontal direction with respect to the gravity direction (vertical downward direction), the steam sufficiently spreads.

Since the upward direction of the tray 35 is clear for the user, the user holds the handles 62 of the tray 35 to move the tray 35 into the chamber 51 as it is. Then, the treatment tool insertion port 25 opening or the opening of the air or water feeding cylinder is naturally directed in the gravity direction (vertical downward direction) or obliquely upwards from the horizontal direction with respect to the gravity direction (vertical downward direction) in the chamber 51 in the autoclave device 50.

It is to be noted that the endoscope 2 and the members and the like detached from the endoscope 2 are directly stored in the chamber 51 in the autoclave sterilization device 50 without using the tray 35. In this case, the endoscope 2 may also be tilted to dispose an installation instrument to be installed in the chamber 51 in such a manner that the treatment tool insertion port 25 opening of the endoscope operation portion 8, the air or water feeding cylinder, the opening of the other end of the channel disposed in the tip portion 17, or at least one opening of each pipe line disposed in the endoscope 2 is directed in the gravity direction (vertical downward direction) even slightly from the horizontal direction or obliquely upwards from the horizontal direction with respect to the gravity direction (vertical downward direction).

Then, the above-described constitution produces the following effect. That is, in the present embodiment, during the cleaning of the endoscope 2 after the end of the endoscopic inspection, the endoscope 2 and the member and the like detached beforehand from the endoscope 2 are stored beforehand in the tray 35. Thereafter, the tray 35 is set in the cleaning bath 41 of the endoscope cleaner 40 and cleaned. After the cleaning, the tray 35 is removed from the cleaning bath 41 of the endoscope cleaner 40, and the tray 35 is stored in the chamber 51 of the autoclave sterilization device 50 still in this state to perform the autoclave sterilization (high temperature and pressure steam sterilization). This saves a trouble of setting the endoscope 2 after cleaned and the member and the like detached from the endoscope 2 anew in the tray or the container before the autoclave.

As a result, in the endoscope cleaning and sterilizing system of the present embodiment, it is possible to save a trouble of setting the endoscope 2 again in the predetermined shape, when the endoscope 2 is transported to the autoclave device 50 from the endoscope cleaner 40. Therefore, when the endoscope 2 used in the endoscopic inspection or the like is cleaned, and thereafter subjected to the autoclave sterilization treatment, the operation is facilitated.

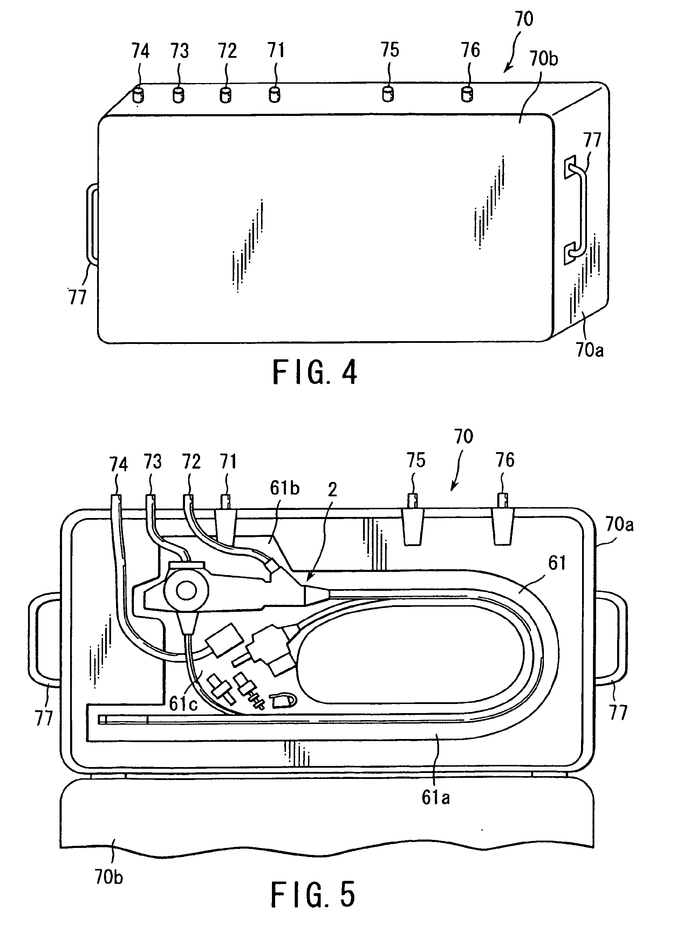

Moreover, FIGS. 4 and 5 show a second embodiment of the present invention. FIG. 4 is a perspective view showing an appearance of a sterilization container according to the second embodiment of the present invention, and FIG. 5 is a plan view showing an opened state of the lid of the sterilization container of FIG. 4.

In the first embodiment, the tray 35 in which the endoscope storage portion 61 is disposed as the concave portion is used as the high temperature and pressure steam sterilization container for the endoscope to constitute the endoscope cleaning and sterilizing system. On the other hand, in the second embodiment, a sterilization container 70 shown in FIG. 4 is used as the high temperature and pressure steam sterilization container for the endoscope to constitute the endoscope cleaning and sterilizing system. Since the other constitution is similar to the above-described first embodiment, the description is omitted, and the same constitution is denoted with the same reference numerals for the description.

As shown in FIG. 4, in the sterilization container 70 which is the high temperature and pressure steam sterilization container for the endoscope, a container main body 70a and a lid 70b are disposed. The container main body 70a is sealed by the lid 70b and constituted so that the steam is capable of entering but the liquid is incapable of entering the container. Therefore, the sterilization container 70 is capable of holding the sterilization in the sealed state as it is after the autoclave sterilization. It is to be noted that the sterilization container 70 is formed of a material having resistance to the high temperature and pressure steam at the autoclave sterilization (high temperature and pressure steam sterilization) time.

As shown in FIG. 5, the endoscope storage portion 61 similar to that of the above-described first embodiment is disposed in the container main body 70a of the sterilization container 70. The endoscope 2 and the member and the like detached from the endoscope 2 are stored in this endoscope storage portion 61.

A water injection cap 71, a treatment channel cleaning cap 72, an air or water feeding cleaning cap 73, and a water discharge cap 74 are protruded from the side wall surface of the container main body 70a. Outer end portions of these water injection cap 71, treatment channel cleaning cap 72, air or water feeding cleaning cap 73, and water discharge cap 74 are detachably connectable to the water injection port 43 of the endoscope cleaning device 40 of the first embodiment, and caps corresponding to the treatment channel cleaning tool 44, air or water feeding cleaning tool 45, and water discharge tool 46.

Inner end portions of these caps are extended into the container main body 70a as shown in FIG. 5, and are shaped in the same manner as in the water injection port 43, treatment channel cleaning tool 44, air or water feeding cleaning tool 45, and water discharge tool 46 of the endoscope cleaning device 40 described in the above-described first embodiment. It is to be noted that, additionally, further cleaning tools for cleaning separate pipe lines and the like may also be disposed in the sterilization container 70 in accordance with the function of the endoscope.

Moreover, a steam injection cap 75 and steam discharge cap 76 for use at an autoclave treatment time are protruded from the side wall surface of the container main body 70a. The outer end portions of these steam injection cap 75 and steam discharge cap 76 are detachably connectable to caps for steam injection and discharge (not shown) disposed in the autoclave device 50.

The inner end portions of these steam injection cap 75 and steam discharge cap 76 have shapes suitable for the steam injection and discharge.

Moreover, handles 77 are disposed on the opposite ends of the container main body 70a. When these handles 77 are grasped, the sterilization container 70 is easily transported.

Next, the function of the present embodiment constituted as described above will be described. In the present embodiment, after the end of the endoscopic inspection, the endoscope 2 and the member and the like detached from the endoscope 2 are stored in the predetermined places of the endoscope storage portion 61 of the sterilization container 70.

Thereafter, the sterilization container 70 is stored in the cleaning bath 41 of the endoscope cleaning device 40. At this time, the sterilization container 70 is pushed and stored into the cleaning bath 41 of the endoscope cleaning device 40. Then, the caps corresponding to the water injection port 43, treatment channel cleaning tool 44, air or water feeding cleaning tool 45, and water discharge tool 46 of the endoscope cleaning device 40 are easily (naturally) connected to the water injection cap 71, treatment channel cleaning cap 72, air or water feeding cleaning cap 73, and water discharge cap 74.

After ending the preparation for the endoscope cleaning in this manner, the user closes the lid 70b, and operates the endoscope cleaning device 40 to clean the endoscope. After ending the cleaning, the user removes the sterilization container 70 from the endoscope cleaning device 40.

Thereafter, the user stores the sterilization container 70 in which the endoscope 2 and the member and the like detached from the endoscope 2 remain to be stored in the chamber 51 in the autoclave device 50. At this time, when the sterilization container 70 is pushed and stored in the chamber 51 in the autoclave device 50, the caps for steam injection and discharge of the autoclave device 50 are easily (naturally) connected to the steam injection cap 75 and steam discharge cap 76. The autoclave sterilization (high temperature and pressure steam sterilization) is performed in this state.

As described above, the sterilization container 70 of the present embodiment itself has a function of cleaning bath 41 in the endoscope cleaning device 40, and also has a function of the chamber 51 in the autoclave device 50. Needless to say, the sterilization container 70 is locked not to break the sealed and closed state of the sterilization container 70 while the cleaning and autoclave sterilization are performed.

Then, the above-described constitution produces the following effect. That is, the sterilization container 70 of the present embodiment is capable of performing the cleaning and sterilizing without being opened, after the endoscope 2 and the member and the like detached from the endoscope 2 are stored in the sterilization container 70. Thereafter, the members can be stored while the sterilization is held. Therefore, when the endoscope 2 used in the endoscopic inspection or the like is cleaned, and thereafter subjected to the autoclave sterilization treatment, the operation is facilitated.

Moreover, for the sterilization container 70 of the present embodiment, the sterilization container 70 itself serves as the chamber 51 in the autoclave device 50. Therefore, since the device can be operated with a minimum steam amount necessary for sterilizing the endoscope 2 and the member and the like detached from the endoscope 2, a series of sterilization cycle can be shortened.

As a result, the endoscope cleaning and sterilizing system of the second embodiment further saves a user's trouble as compared with the above-described first embodiment.

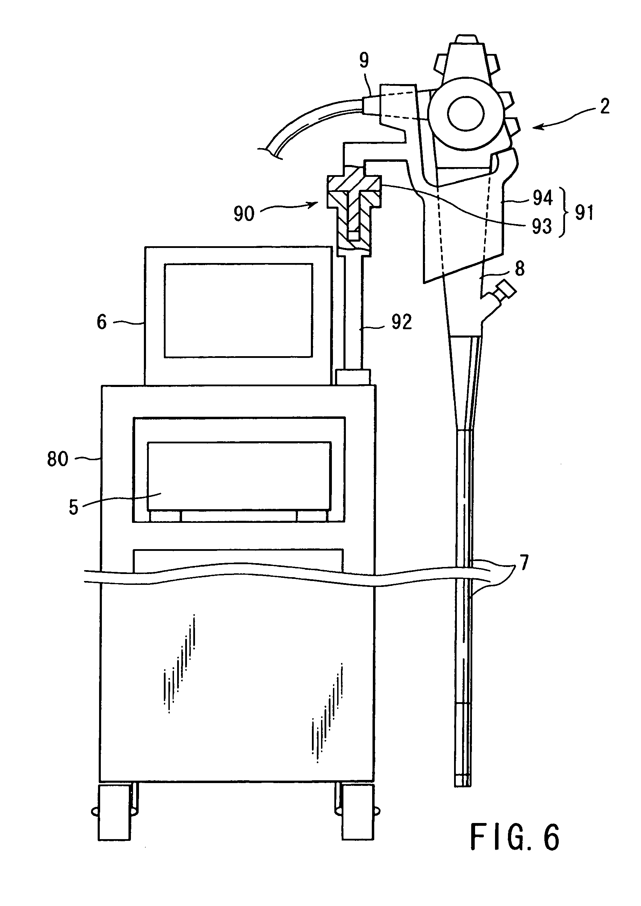

Moreover, FIG. 6 is an explanatory view showing a hanger device disposed in a trolley 80.

As shown in FIG. 6, apparatuses necessary for constructing an endoscope image, such as the video processor 5 and monitor 6, are stored in the trolley 80. A hanger 90 from which the endoscope 2 is hung on standby immediately before the inspection is attached to a part of the trolley 80.

The hanger 90 is constituted of a head portion 91 and a base portion 92. The head portion 91 is constituted of a connecting portion 93 and an endoscope attaching portion 94. This head portion 91 has a resistance to the autoclave sterilization. The connecting portion 93 of the head portion 91 is detachably attached to the base portion 92.

For the hanger 90 constituted in this manner, the endoscope attaching portion 94 is detached from the base portion 92 together with the endoscope 2 after the endoscopic inspection, cleaned, and autoclaved and sterilized. Furthermore, after the sterilization, the endoscope attaching portion 94 attached to the endoscope 2 is connected to the base portion 92.

Additionally, in general, after the sterilization, the endoscope 2 is hung from the hanger on standby immediately before the inspection in many cases. When this sterilized endoscope 2 is hung, there is also a possibility of contamination by the hanger. The hanger has not heretofore been autoclaved and sterilized together with the endoscope which can be autoclaved and sterilized.

Then, for the hanger 90 of FIG. 6, when the sterilized endoscope 2 is on standby immediately before the inspection, the hanger 90 from which the endoscope 2 is to be hung is constituted in such a manner that the head portion 91 may be autoclaved and sterilized together with the endoscope 2 so as to prevent the contamination by the hanger 90.

Moreover, in the endoscopic inspection, the endoscope 2 is detached from the endoscope attaching portion 94 and used. In this manner, the endoscope 2 (operation portion 8 or insertion portion 7) does not contact the member which has not been autoclaved and sterilized until the endoscopic inspection.

As a result, the hanger 90 from which the endoscope 2 is to be hung can be autoclaved and sterilized together with the endoscope 2, and accordingly there is not a possibility that the endoscope 2 on standby is contaminated immediately before the inspection.

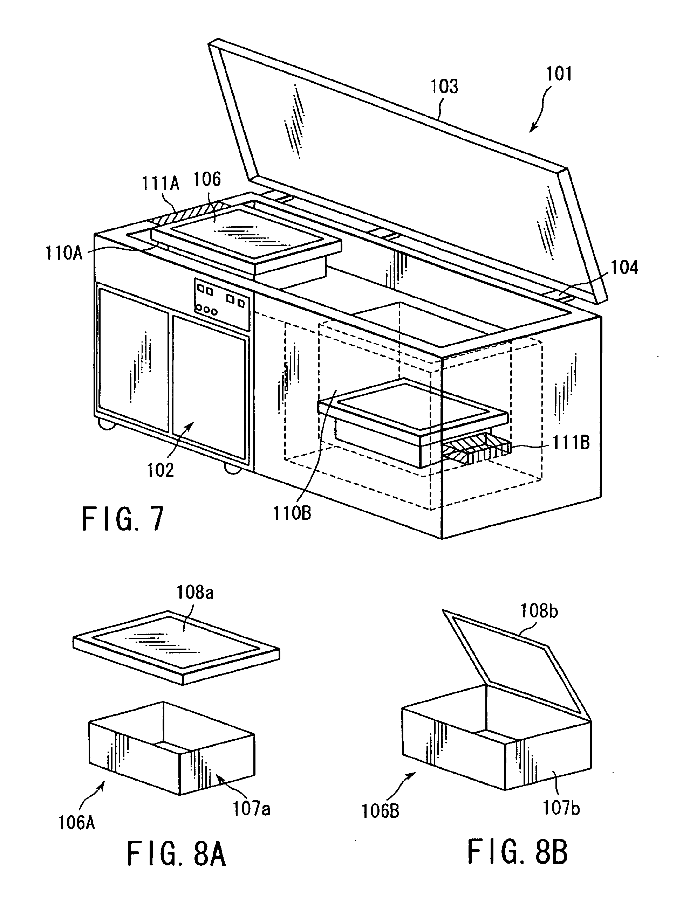

Next, an example, not forming part of the claimed invention will be described with reference to FIG. 7 to FIGS. 14A to 14C. FIG. 7 shows the appearance of an endoscope cleaning, disinfecting and sterilizing device 101 which is a reproducing treatment device of the used endoscope of the present embodiment. This endoscope cleaning, disinfecting and sterilizing device 101 is provided with a device main body 102 having a substantial box shape. An openable and closable lid body 103 is disposed on the upper surface of the device main body 102. This lid body 103 is rotatably connected to a back wall portion of the device main body 102 via a hinge portion 104.

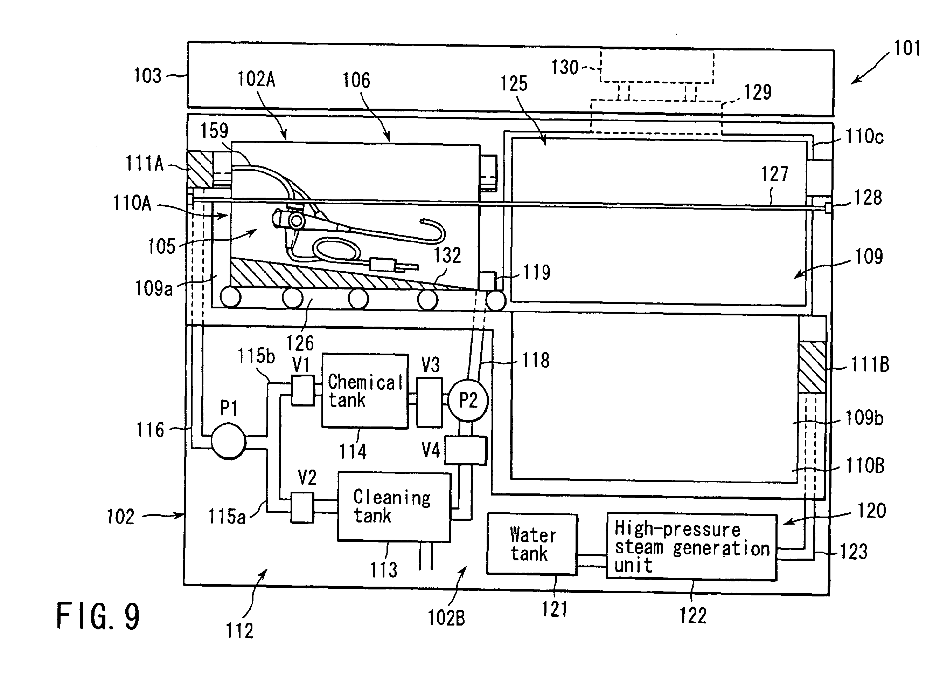

Moreover, as shown in FIG. 9, a substantially L-shaped endoscope treatment chamber 102A is disposed on an upper-part side, and a machine chamber 102B is disposed on a lower side of the endoscope treatment chamber 102A in the device main body 102. Furthermore, a tray 106 is disposed in the endoscope treatment chamber 102A. Materials to be cleaned and sterilized such as an endoscope 105 which is a treatment object are stored in the tray 106.

The tray 106 includes a tray 106A of a lid separated type as shown in FIG. 8A, and a tray 106B of a lid integral structure type as shown in FIG. 8B. Here, in the tray 106A of the lid separated type, a tray main body 107a having a substantial box shape whose upper surface is opened, and a tray lid 108a having the separated structure are combined and constituted. The upper surface opening of the tray main body 107a is opened and closed by the tray lid 108a having the separated structure.

Furthermore, one side portion of a tray lid 108b is rotatably connected and constituted to one side portion of the upper surface opening of a tray main body 107b having the substantial box shape whose upper surface is opened via a hinge portion in the tray 106B of the lid integral structure type. Moreover, either of the tray 106A of the lid separated type or the tray 106B of the lid integral structure type may be used in the tray 106.

Moreover, a tray moving path 109 bent in a substantial L shape is disposed in the endoscope treatment chamber 102A of the device main body 102. This tray moving path 109 is constituted of a transverse moving path 109a and a vertical moving path 109b. The transverse moving path 109a holds the tray 106 so that the tray is movable in a transverse direction (right or left direction) in FIG. 9. The vertical moving path 109b holds the tray 106 so that the tray is movable in a longitudinal direction (vertical direction) on a right end portion side of the transverse moving path 109a in FIG. 9.

Here, a first treatment section (first treatment means) 110A is formed in a left-side portion (side portion of the vertical moving path 109b) in FIG. 9 in the transverse moving path 109a. The first treatment section 110A cleans or cleans and disinfects the endoscope 105 in the tray 106. A tray connecting portion 111A is disposed in the side wall portion of the first treatment section 110A. The tray connecting portion 111A is detachably connected to the tray 106.

Furthermore, a second treatment section (second treatment means) 110B is disposed in a portion of the vertical moving path 109b below the transverse moving path 109a in FIG. 9. The second treatment section 110B is a chamber for sterilization, which sterilizes the endoscope 105 cleaned or cleaned and disinfected in the first treatment section 110A.

A tray connecting portion 111B and a discharge portion (not shown) are disposed in the side wall portion of the second treatment section 110B. The tray connecting portion 111B is detachably connected to the tray 106. The discharge port (not shown) is constituted in the same manner as in a discharge port 119 of the first treatment section 110A. Moreover, this discharge port is detachably connected to a solution discharge valve 133 of the tray 106. It is to be noted that the discharge port 119 of the second treatment section 110B is connected to a discharge pipe line (not shown).

Moreover, a first treatment unit 112 is disposed in a portion under the transverse moving path 109a in the first treatment section 110A. A cleaning tank 113 and a chemical tank 114 are disposed in the first treatment unit 112. A cleaning solution for use in the cleaning process of the first treatment section 110A is stored in the cleaning tank 113. An antiseptic solution for use in the disinfecting process of the first treatment section 110A is stored in the chemical tank 114. Moreover, this first treatment unit 112 is used in cleaning or cleaning and disinfecting the endoscope 105 in the tray 106.

Furthermore, fluid pipe lines 115a, 115b connected in parallel with each other are disposed in the first treatment unit 112. Moreover, one fluid pipe line 115a is provided with the cleaning tank 113, and the other fluid pipe line 115b is provided with the chemical tank 114. Here, the cleaning tank 113 on a downstream side in the fluid pipe line 115a is connected to a pump P1 on a suction port side via an electromagnetic valve V2. It is to be noted that the chemical tank 114 on the downstream side in the fluid pipe line 115b is combined with the fluid pipe line 115a via an electromagnetic valve V1. This combined portion is connected to the common pump P1 on the suction port side.

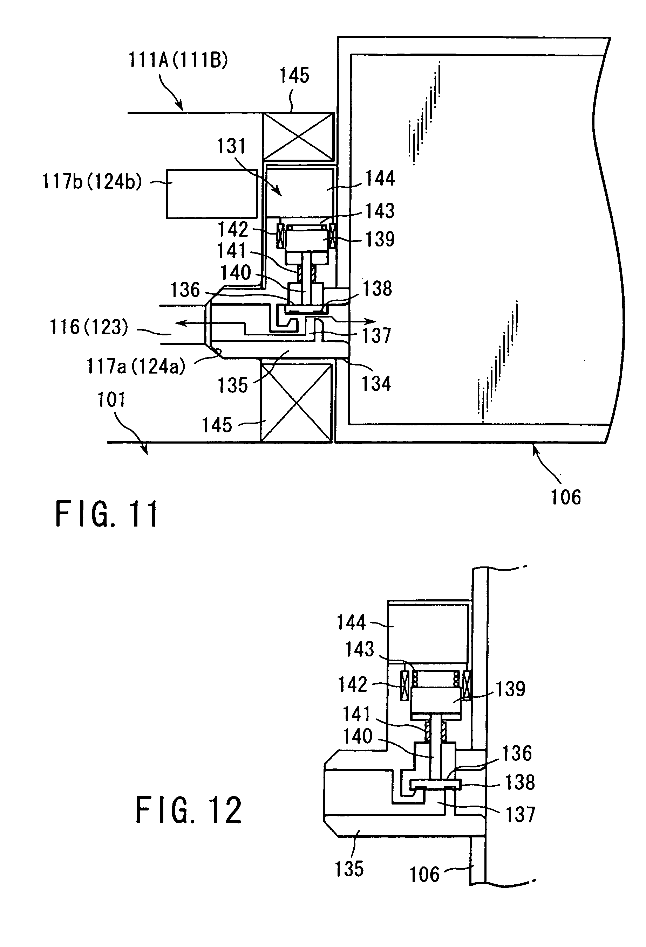

Moreover, the pump P1 on a discharge port side is connected to an inlet portion of a supply pipe line 116. Here, as shown in FIG. 11, a tray side pipe line connecting portion 117a and a first main body side transmission and reception unit 117b are disposed in the tray connecting portion 111A of the first treatment section 110A of the device main body 102. The first main body side transmission and reception unit 117b controls the opening and closing of valves for opening and closing the pipe lines 131a, 131b described later in the tray 106. Moreover, the tray side pipe line connecting portion 117a of the tray connecting portion 111A is connected to an outlet portion of the supply pipe line 116.

Furthermore, the cleaning tank 113 on an upstream side in the fluid pipe line 115a is connected to a pump P2 on a discharge port side via an electromagnetic valve V4. It is to be noted that the chemical tank 114 on the upstream side in the fluid pipe line 115b is connected to the pump P2 common to the fluid pipe line 115a on the discharge port side via an electromagnetic valve V3. This pump P2 on the suction port side is connected to one end of a discharge pipe line 118. The other end of the discharge pipe line 118 is connected to the discharge port 119 of the first treatment section 110A.

Moreover, a second treatment unit 120 is disposed in a part under the second treatment section 110B. The second treatment unit 120 is a sterilization unit for performing a high-pressure steam sterilization with respect to the endoscope 105 in the tray 106. This second treatment unit 120 is provided with a water tank 121 for autoclave and a high-pressure steam generation unit 122. Moreover, a high-pressure steam for the autoclave is generated in the high-pressure steam generation unit 122 with respect to the water for the autoclave supplied to the high-pressure steam generation unit 122 from the water tank 121 for autoclave.

Furthermore, the high-pressure steam generation unit 122 is connected to one end of a high-pressure steam supply pipe line 123. Here, the tray connecting portion 111B of the second treatment section 110B is provided with a tray-side pipe line connecting portion 124a and a second main body side transmission and reception unit 124b in the same manner as in the tray connecting portion 11.1A of the first treatment section 110A. The second main body side transmission and reception unit 124b controls the opening and closing of valves 131c, 131d for opening and closing the pipe lines described later on the side of the chamber for sterilization disposed in the tray 106. Moreover, the tray-side pipe line connecting portion 124a of the tray connecting portion 111B is connected to the other end of the high-pressure steam supply pipe line 123.

It is to be noted that a chamber lid 110C is disposed above the second treatment section 110B. The chamber lid 110C has a structure resistant to the high pressure in the chamber of the second treatment section 110B at the sterilization time. Moreover, the chamber lid 110C is constituted to be automatically openable and closable by control means of the device main body 102 of the endoscope cleaning, disinfecting and sterilizing device 101.

Moreover, tray conveying means 125 is disposed in the device main body 102. The tray conveying means 125 conveys the tray 106 between the first treatment section 110A and the second treatment section 110B. The tray conveying means 125 is provided with a belt conveyor 126 and a rail 127 for conveying the tray. The belt conveyor 126 is disposed on the floor surface of the transverse moving path 109a. The rail 127 for conveying the tray is extended along a conveying direction in the transverse direction of the tray 106 in the inner wall surface of the device main body 102 to control a conveying position of the tray 106. Furthermore, a rail control unit 128 is disposed in a terminal end portion of the rail 127 for conveying the tray. The rail control unit 128 detaches the tray 106 from the rail 127 for conveying the tray.

Moreover, an adsorption member 129 and a motor 130 for lifting up and down are disposed in a ceiling part of the vertical moving path 109b. The adsorption member 129 temporarily holds the tray 106 in an upper part of the second treatment section 110B which is the chamber for sterilization. The motor 130 for lifting up and down generates a driving force for moving down the tray 106 temporarily held by the adsorption member 129 in the second treatment section 110B.

Furthermore, the tray 106 is temporarily held by the adsorption member 129 in the connecting portion between the transverse moving path 109a and the vertical moving path 109b at the moving time of the tray 106. In this state, the tray 106 is detached from the rail 127 for conveying the tray by the rail control unit 128. Furthermore, in this state, the tray 106 temporarily held by the adsorption member 129 is moved down into the second treatment section 110B by the motor 130 for lifting up and down. Therefore, when the cleaning and disinfecting process is completed in the first treatment section 110A, the tray 106 is conveyed into the second treatment section 110B which is the chamber for sterilization by the conveying means 125.

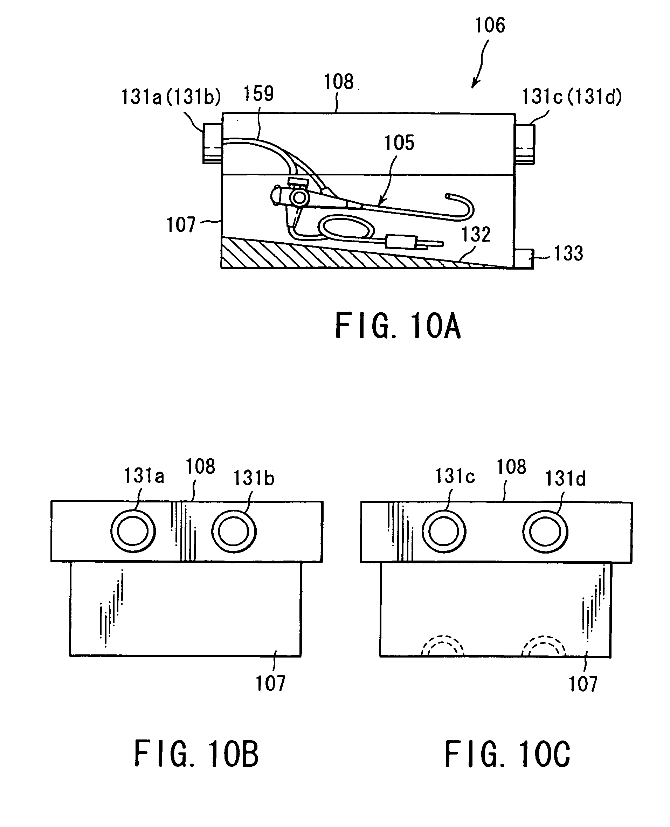

Moreover, as shown in FIG. 10A, a plurality of valves 131a to 131d for opening and closing the pipe lines are disposed in the opposite side surfaces of a tray lid 108 of the tray 106. Here, as shown in FIG. 10B, two valves 131a, 131b for opening and closing the pipe lines are disposed in one side surface of the tray lid 108. As shown in FIG. 10C, two valves 131c, 131d for opening and closing the pipe lines are similarly disposed in the other side surface of the tray lid 108. It is to be noted that these valves 131a to 131d for opening and closing the pipe lines may also be disposed in the opposite side surfaces of the tray main body 7.

Furthermore, an inclined surface 132 inclined at an appropriate angle with respect to a horizontal plane is formed in a bottom part of the tray 106. A solution discharge valve 133 is disposed in a lowermost part of the inclined surface 132. The solution discharge valve 133 discharges a waste solution while the cleaning and disinfecting is performed in the tray 106. It is to be noted that the solution discharge valve 133 of the tray 106 is structured in the same manner as in the valves 131a, 131b for opening and closing the pipe lines. Moreover, the waste solution and the like in the tray 106 can be securely discharged from the solution discharge valve 133.

Moreover, FIG. 11 shows the inner structures of the respective valves 131a to 131d for opening and closing the pipe lines of the tray 106. It is to be noted that basic structures of the valves 131a to 131d for opening and closing the pipe lines are modifications of the structure of the electromagnetic valve, and all the valves have the same structure. Moreover, here the constitution of one valve 131 for opening and closing the pipe line will be described.

In FIG. 11, reference numeral 134 denotes an opening for attaching the valve, formed in one side part of the tray 106. This opening 134 is connected to a pipe line constituting member 135 of the valve 131 for opening and closing the pipe line. A valve body 136 of the valve 131 for opening and closing the pipe line for opening and closing this pipe line is disposed midway in the pipe line of the pipe line constituting member 135. This valve body 136 is movably supported in a direction (opening and closing direction) crossing a center line direction of the pipe line constituting member 135 at right angles. Furthermore, a communicating port 137 is disposed in the pipe line of the pipe line constituting member 135. The communicating port 137 is opened and closed by the valve body 136 of the valve 131 for opening and closing the pipe line.

Moreover, a packing 138 for securely opening and closing the valve 131 is disposed in the valve body 136 of the valve 131 for opening and closing the pipe line. Furthermore, a plunger 139 for moving and operating the valve body 136 in the opening and closing direction is disposed in the valve 131 for opening and closing the pipe line. A sealing material 141 is disposed around a shaft 140 of the plunger 139. Furthermore, a coil 142 is disposed on an outer periphery of the plunger 139.

Furthermore, a coil spring 143 is disposed in the upper part of the plunger 139. Moreover, usually (at a time when power is not supplied to the coil 142), as shown in FIG. 12, the valve body 136 of the valve 131 for opening and closing the pipe line is allowed to abut on a valve seat portion of a peripheral edge of the communicating port 137 and urged in a direction for closing the communicating port 137 by a spring force of the coil spring 143. This achieves a structure in which the pipe line of the pipe line constituting member 135 is blocked midway.

Moreover, the valve 131 for opening and closing the pipe line is integrated with a tray side transmission and reception unit 144 on which means for driving the coil 142 is mounted. FIG. 9 shows a state in which the tray 106 is disposed in the first treatment section 110A of the device main body 102, and the supply pipe line 116 of the tray side pipe line connecting portion 117a in the tray connecting portion 111A of the first treatment section 110A is connected to the pipe line of the pipe line constituting member 135 of the tray 106. In this state, in FIG. 10A, each tray side transmission and reception unit 144 for two valves 131a, 131b for opening and closing the pipe lines in the left-side surface of the tray 106 is disposed in a position corresponding to the first main body side transmission and reception unit 117b of the device main body 102. Moreover, the tray side transmission and reception unit 144 on the side of the tray 106 is controlled by the first main body side transmission and reception unit 117b. At this time, the coil 142 of the valve 131 for opening and closing the pipe line is driven by the tray side transmission and reception unit 144 to control the opening and closing operations of the respective valves 131a, 131b for opening and closing the pipe lines.

Here, when the coil 142 is driven, the plunger 139 is moved in an adsorbed direction on the coil 142 side against the spring force of the coil spring 143. With this operation, as shown in FIG. 11, the valve body 136 of the valve 131 for opening and closing the pipe line is detached from the valve seat portion of the peripheral edge of the communicating port 137. As a result, the communicating port 137 is opened to release the pipe line of the pipe line constituting member 135. In this state, the cleaning solution or antiseptic solution is supplied into the tray 106 of the first treatment section 110A to clean and disinfect the endoscope 105 in the tray 106.

Moreover, one of the valves 131a, 131b for opening and closing the pipe lines of the tray 106 connected to the tray connecting portion 111A of the first treatment section 110A is connected to one end of a cleaning and disinfecting tube 159. The other end of the cleaning and disinfecting tube 159 is connected to various channels of the endoscope 105. Moreover, the insides of various channels of the endoscope 105 can be cleaned securely at the cleaning and disinfecting time of the endoscope 105 in the first treatment section 110A.

Furthermore, the solution discharge valve 133 of the tray 106 is connected to the discharge port 119 of the first treatment section 110A in a state in which the tray connecting portion 111A of the first treatment section 110A is connected to the tray 106. At this time, the discharge port 119 of the first treatment section 110A is connected to the discharge pipe line 118. Moreover, the waste solution during the cleaning and disinfecting of the endoscope 105 in the tray 106 in the first treatment section 110A is discharged to the discharge pipe line 118 from the solution discharge valve 133 via the discharge port 119 of the first treatment section 110A.

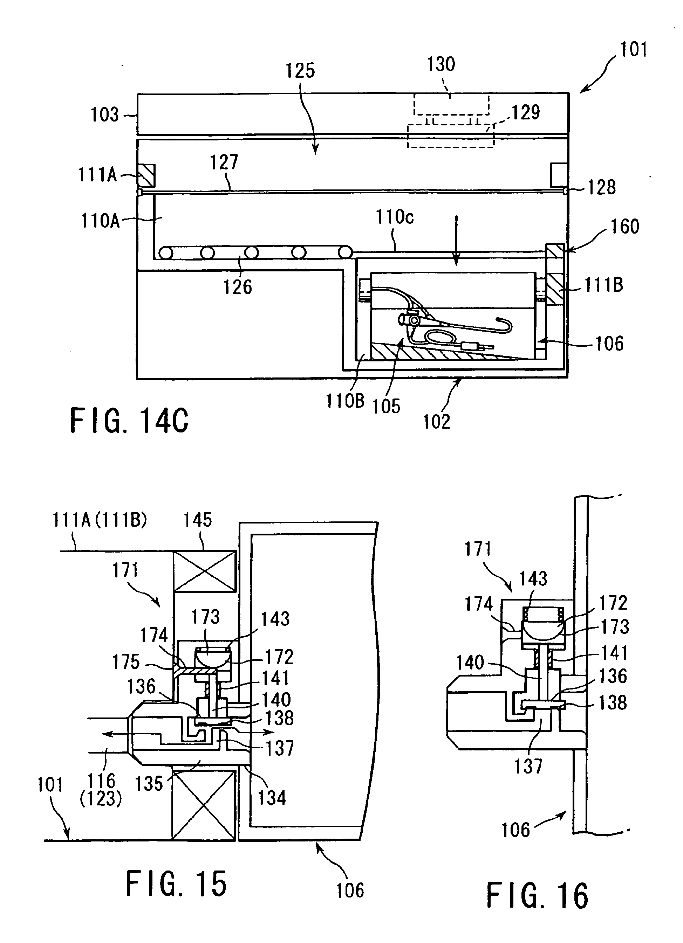

Moreover, FIG. 14C shows a state in which the tray 106 moves to the second treatment section 110B. At this time, the high-pressure steam supply pipe line 123 of the tray-side pipe line connecting portion 124a in the tray connecting portion 111B is connected to the pipe line of the pipe line constituting member 135 of the tray 106. In this state, in FIG. 10A, each tray side transmission and reception unit 144 for two valves 131c, 131d for opening and closing the pipe lines in the right-side surface of the tray 106 is disposed in the position corresponding to the second main body side transmission and reception unit 124b of the device main body 102. Moreover, the tray side transmission and reception unit 144 on the tray 106 side is controlled by the second main body side transmission and reception unit 124b of the device main body 102, and the opening and closing operations of the respective valves 131c, 131d for opening and closing the pipe lines are controlled.

Moreover, the tray side transmission and reception unit 144 of the tray 106 transmits and receives power and data with respect to the first main body side transmission and reception unit 117b and second main body side transmission and reception unit 124b of the device main body 102 by a method without any electric contact. Accordingly, a driven state of each valve 131 and an operation state of each valve 131 can be detected.

A basic principle of the method of transmission and reception without any electric contact is application of radio frequency identification (RFID) using radio waves. Here, the RFID generally includes "electromagnetic coupling system", "electrostatic coupling system", "electromagnetic inductive system", "microwave system", and "optical communication system". As the application of the present example, the "electromagnetic coupling system", "electromagnetic inductive system", and "microwave system" are capable of fulfilling more effect. It is to be noted that the application is possible even in another case.

Moreover, in the present example, the tray connecting portion 111A of the first treatment section 110A in the device main body 102 and the tray connecting portion 111B of the second treatment section 110B are provided with adsorption members 145 such as an electromagnet and suction unit. Moreover, as shown in FIG. 11, the adsorption member 145 adsorbs the tray 106 in a state in which the tray 106 is disposed in the first treatment section 110A. Accordingly, the tray is held in a state in which the first main body side transmission and reception unit 117b is securely connected to the tray side transmission and reception unit 144. Similarly, the adsorption member 145 adsorbs the tray 106 in a state in which the tray 106 is disposed in the second treatment section 110B. Accordingly, the tray is held in a state in which the second main body side transmission and reception unit 124b is securely connected to the tray side transmission and reception unit 144. It is to be noted that the electromagnet, suction method by a vacuum pump or the like, or mechanical holding means may also be used in the adsorption member 145.

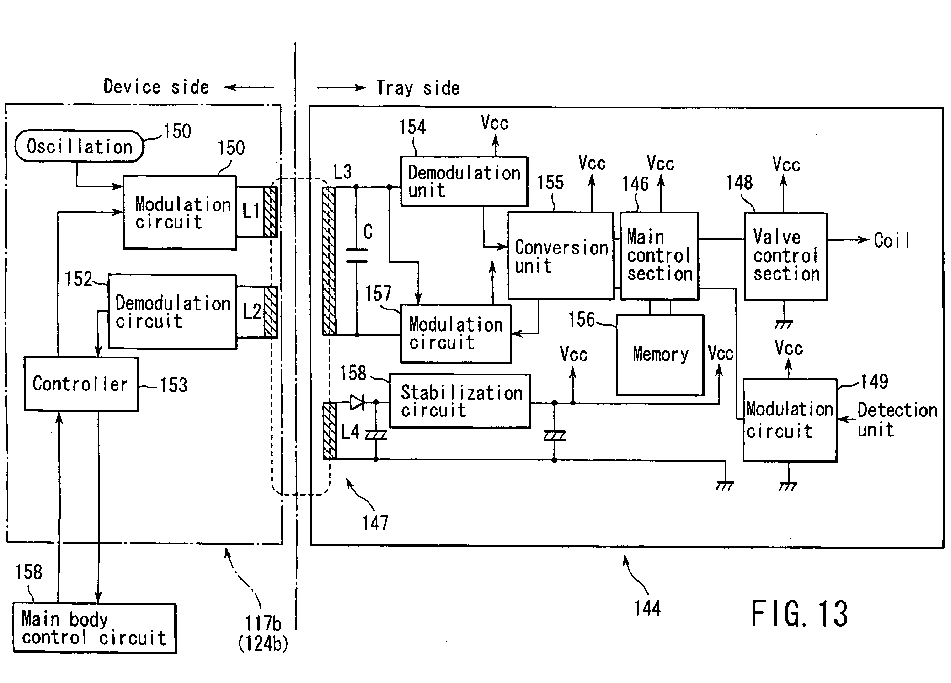

Moreover, FIG. 13 shows an electric circuit of the tray side transmission and reception unit 144 and the first main body side transmission and reception unit 117b (second main body side transmission and reception unit 124b) in the endoscope cleaning, disinfecting and sterilizing device 101. Here, the tray side transmission and reception unit 144 is provided with main control sections 146 such as CPU, a transmission and reception circuit 147, a power supply circuit, a valve control section 148, and a conversion circuit 149 for converting the signal of a detection unit (not shown) disposed in the valve 131 for opening and closing the pipe line into a digital signal or the like.

Furthermore, the first main body side transmission and reception unit 117b (second main body side transmission and reception unit 124b) is provided with a modulation circuit 150 for preparing and transmitting the transmission signal to the tray side transmission and reception unit 144, a transmission coil L1, and an oscillation unit 151. Furthermore, a reception coil L2 for receiving the signal from the tray side transmission and reception unit 144, a demodulation circuit 152, and a controller 153 for controlling the constitution are disposed. The controller 153 is connected to a main body control circuit 158 having a function of controlling various units.

Furthermore, on the tray side transmission and reception unit 144 side, there are disposed: a coil L3 for transmitting and receiving the signal; a capacitor C for resonance; a demodulation unit 154 for demodulating a reception signal; a conversion unit 155; a memory 156 in which data of the conversion unit 155 is stored; a main control section 146 for controlling the conversion unit 155 and memory 156 to control communication with the main body; and a modulation circuit 157 for the transmission. Moreover, the signal received by a coil L4 is smoothed and rectified by a stabilization circuit 158 and supplied to each circuit to achieve the power supply for driving these circuits.

Next, the function of the above-described constitution will be described. The endoscope cleaning, disinfecting and sterilizing device 101 of the present example is used after the end of the diagnosis by the endoscope 105. Here, after the diagnosis by the endoscope 105 ends, the used endoscope 105 is set in the endoscope cleaning, disinfecting and sterilizing device 101. At this time, the endoscope 105 is disposed in the tray 106. Subsequently, the cleaning and disinfecting tube 159 is connected to the endoscope 105.

Thereafter, the tray 106 is set in the first treatment section 110A to clean or clean and disinfect the endoscope 105 in a state in which the lid 108 of the tray 106 is closed. At this time, the supply pipe line 116 of the tray side pipe line connecting portion 117a in the tray connecting portion 111A of the first treatment section 110A is connected to the pipe line of the pipe line constituting member 135 of two valves 131a, 131b for opening and closing the pipe lines on the left-side surface of the tray 106.

Furthermore, as shown in FIG. 11, the tray 106 is adsorbed by the adsorption member 145 in a state in which the tray 106 is disposed in the first treatment section 110A. Accordingly, the first main body side transmission and reception unit 117b is securely connected to the tray side transmission and reception unit 144. When the power of the device 101 is turned on in this state, an initial state is set, and the cleaning process is automatically started.

Moreover, at an on-operation time of the power supply of the device main body 102, the radio frequency identification (RFID) system automatically operates by the control signal from the main body control circuit 158 in the device main body 102. At this time, data communication is performed between the tray side transmission and reception unit 144 of the tray 106 and the first main body side transmission and reception unit 117b.

At a data write operation time, a radio frequency signal digital-modulated by the modulation circuit 150 is applied to the transmission coil L1 in accordance with write command and write information transmitted as serial signals from the controller 153 of the first main body side transmission and reception unit 117b. At this time, the signal induced in the coil L3 of the tray side transmission and reception unit 144 is amplified, and demodulated to an original digital signal in the demodulation unit 154.

Subsequently, digitized serial signal information is converted to a parallel signal by the conversion unit 155, and the information is stored in the data memory 156. Furthermore, the control signal is transferred to the valve control section 148 by the signal from the main control section 146. Moreover, the coil 142 of the valve 131 for opening and closing the pipe line is driven by the control signal output from the valve control section 148. At this time, the valves 131a, 131b for opening and closing the pipe lines are released to secure the pipe line for the cleaning and disinfecting. In this state, the cleaning solution or the antiseptic solution is supplied into the tray 106 of the first treatment section 110A to clean and disinfect the endoscope 105 in the tray 106. At this time, the cleaning solution or the antiseptic solution is supplied into various channels of the endoscope 105 from the cleaning and disinfecting tube 159 to securely clean the inside of various channels of the endoscope 105.

Furthermore, the waste solution during the cleaning and disinfecting of the endoscope 105 in the tray 106 by the first treatment section 110A is discharged to the discharge pipe line 118 from the solution discharge valve 133 via the discharge port 119 of the first treatment section 110A.

Moreover, read operation information (data) of the valves 131a, 131b for opening and closing the pipe lines are transmitted to the main control section 146 and memory 156 from the detection unit via the conversion circuit 149. Here, the parallel information read from the memory 156 is converted into the serial signal in synchronization with a non-modulated signal sent from the transmission coil L1. Furthermore, it is controlled whether or not the coil L3 and the tank circuit by the capacitor C for resonance are brought into a resonance state in response to the signal in the modulation circuit 157. Moreover, it is detected whether or not to resonate the tank circuit of the tray side transmission and reception unit 144 via the reception coil L2 on the controller 153 side of the first main body side transmission and reception unit 117b to exchange the information.

Moreover, electromagnetic valves V2, V4 are switched and operated into the open state, electromagnetic valves V1, V3 are switched and operated into the closed state, and the pumps P1, P2 are driven at the start time of the cleaning process of the endoscope 105 in the tray 106 in the first treatment section 110A. At the driving time of these pumps P1 and P2, the cleaning section sent from the cleaning tank 113 is supplied into the tray 106 from one fluid pipe line 115a and supply pipe line 116 via various pipe lines such as the tray side pipe line connecting portion 117a in the tray connecting portion 111A of the first treatment section 110A to clean the endoscope 105 in the tray 106. At this time, a part of the cleaning section flows into various channels of the endoscope 105 through the cleaning and disinfecting tube 159 to clean the inside of various channels of the endoscope 105.

Furthermore, the solution discharge valve 133 of the tray 106 connected to the discharge port 119 of the first treatment section 110A is held in the open state during the cleaning process of the endoscope 105. Therefore, the waste solution in the tray 106 is returned into the cleaning tank 113 via the discharge pipe line 118. Therefore, the cleaning section is circulated in the above-described path to clean the endoscope 105 in the tray 106 and the inside of the various channels of the endoscope 105 during the cleaning process.

Moreover, the driving of the pump P1 is stopped after elapse of a defined time of the cleaning process. Moreover, while the cleaning section in the tray 106 is returned into the cleaning tank 113 via the discharge pipe line 118 by the pump P2, the cleaning section is recovered, and the cleaning process ends. It is to be noted that the present example includes a constitution in which the cleaning section is recovered, but a pipe line for discharging the cleaning section to the discharge port of facilities may also be disposed to discharge the solution to the outside of the device.

Moreover, when the cleaning process ends, the electromagnetic valves V2, V4 shown in FIG. 9 are closed, and the electromagnetic valves V1, V3 are opened. In this state, the pumps P1, P2 are driven to start the disinfecting process. In the disinfecting process, the antiseptic solution sent from the chemical tank 114 is supplied into the tray 106 from one fluid pipe line 115b and supply pipe line 116 via various pipe lines such as the tray side pipe line connecting portion 117a in the tray connecting portion 111A of the first treatment section 110A to disinfect the endoscope 105 in the tray 106. At this time, a part of the antiseptic solution flows into various channels of the endoscope 105 through the cleaning and disinfecting tube 159 to disinfect the inside of various channels of the endoscope 105.

Furthermore, the solution discharge valve 133 of the tray 106 connected to the discharge port 119 of the first treatment section 110A is held in the open state during the disinfecting process of the endoscope 105. Therefore, the waste solution in the tray 106 is returned into the chemical tank 114 via the discharge pipe line 118. Therefore, the antiseptic solution is circulated in the above-described path to disinfect the endoscope 105 in the tray 106 and the inside of the various channels of the endoscope 105 during the disinfecting process of the endoscope 105. It is to be noted that an immersed state is sometimes temporarily held without circulating the antiseptic solution by a medicinal effect of the chemical.

Moreover, the driving of the pump P1 is stopped after the elapse of the defined time of the disinfecting process. Moreover, while the antiseptic solution in the tray 106 is returned into the chemical tank 114 via the discharge pipe line 118 by the pump P2, the antiseptic solution is recovered, and the disinfecting process ends. It is to be noted that a rinsing process may also be performed after the disinfecting process.

Furthermore, the data communication is started again between the tray side transmission and reception unit 144 of the tray 106 and the first main body side transmission and reception unit 117b at the time of the end of the disinfecting process. Accordingly, the tray side transmission and reception unit 144 closes the valves 131a, 131b for opening and closing the pipe lines, and also closes the pipe line of the cleaning and disinfecting tube 159. At this time, the solution discharge pipe line between the solution discharge valve 133 of the tray 106 and the discharge port 119 of the first treatment section 110A also closes. Therefore, the tray 106 is sealed in a state in which the endoscope 105 is stored, and the endoscope 105 is prevented from being contaminated from the outside of the tray 106.

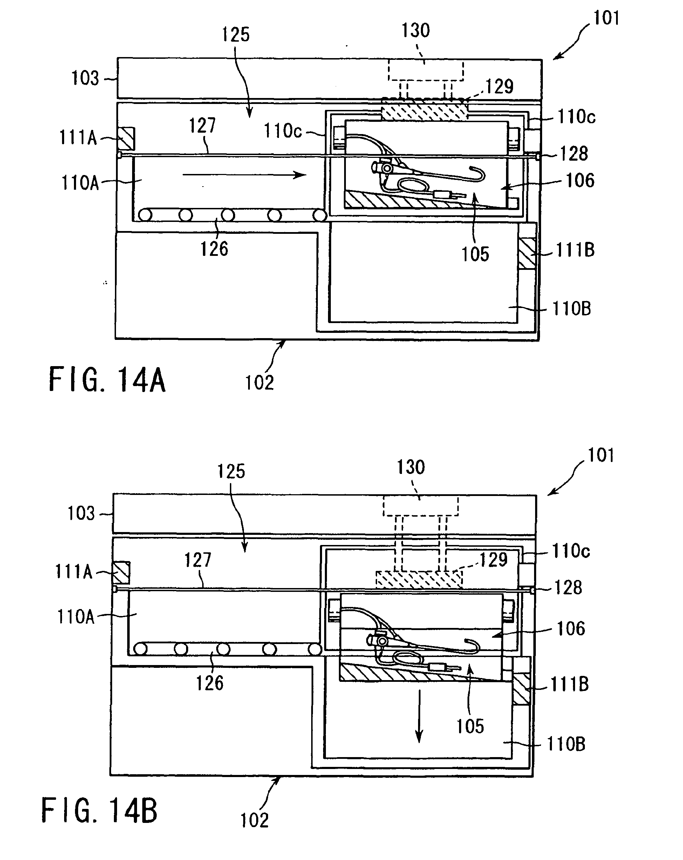

Moreover, after the end of the disinfecting process, the tray 106 kept in the sealed state is conveyed on the right side by the belt conveyor 126 and rail 127 for conveying the tray constituting the tray conveying means 125 in FIG. 9. Moreover, as shown in FIG. 14A, the tray 106 is conveyed to an upper part position of the second treatment section 110B. It is to be noted that, as shown in FIG. 14A, when the tray 106 is conveyed to the upper part position of the second treatment section 110B, the lid for the chamber 110C is in the opened state.

Moreover, when the tray 106 is conveyed to the upper part position of the second treatment section 110B, the tray 106 is adsorbed by the adsorption member 129, the rail control unit 128 operates, and the tray 106 is released from the rail 127 for conveying the tray.

Subsequently, the motor 130 for lifting up and down operates. As shown in FIG. 14B, the tray 106 adsorbed by the adsorption member 129 moves downward in the vertical moving path 109b and is conveyed into the second treatment section 110B. Moreover, the tray 106 is set in a predetermined set position inside the second treatment section 110B. At this time, the high-pressure steam supply pipe line 123 of the tray-side pipe line connecting portion 124a in the tray connecting portion 111B of the second treatment section 110B is connected to the pipe line of the pipe line constituting member 135 of two valves 131c, 131d for opening and closing the pipe lines on the right-side surface of the tray 106.

Furthermore, the second main body side transmission and reception unit 124b is securely connected to the tray side transmission and reception unit 144 in a state in which the tray 106 is disposed in the second treatment section 110B. At this time, the solution discharge valve 133 of the tray 106 is connected to the discharge port of the second treatment section 110B, and the discharge port of the second treatment section 110B is connected to the discharge pipe line.

Moreover, when the tray 106 is set in the set position in the second treatment section 110B, the adsorption member 129 operates, and the tray 106 is released from the adsorption member 129. Thereafter, as shown in FIG. 14C, the chamber lid 110C closes, and the preparation for the sterilizing process in the second treatment section 110B is completed. Here, the chamber lid 110C is fixed by a lid fixing member 60 for the chamber in a state in which the lid is capable of securely bearing the high pressure.

The next sterilizing process is started in this state. At this time, the data communication is started between the tray side transmission and reception unit 144 of the tray 106 and the second main body side transmission and reception unit 124b, and the valves 131c, 131d for opening and closing the pipe lines are released by the tray side transmission and reception unit 144. During the sterilizing process, the solution discharge valve 133 of the tray 106 is controlled in the closed state.

Next, the water in the water tank 121 for autoclave is supplied to the high-pressure steam generation unit 122, and is converted to the high-pressure steam by the high-pressure steam generation unit 122. Moreover, the high-pressure steam supplied from the high-pressure steam generation unit 122 is supplied into the tray 106 of the second treatment section 110B via the high-pressure steam supply pipe line 123, and the endoscope 105 in the tray 106 and the inside of various channels of the endoscope 105 are subjected to the sterilization treatment for the defined time. When the sterilization treatment is completed, the solution discharge valve 133 is released, and the high-pressure steam used in the sterilization treatment is discharged to the solution discharge pipe line via the solution discharge valve 133 from the tray 106, and thereafter discharged to an outside discharge port.

Moreover, when the sterilization treatment is completed, the tray 106 is removed from the second treatment section 110B and conveyed to the initial first treatment section 110A by the tray conveying means 125, and the endoscope 105 in the tray 106 is dried. Here, a series of the respective processes of the cleaning and disinfecting and sterilizing treatment of the endoscope 105 in the curved portion 16 are all completed. It is to be noted that although not shown, the vacuum pump or drying means by hot air is incorporated in the present device, and a drying process may also be incorporated before or after the sterilization treatment.

Then, the above-described constitution produces the following effect. That is, in the endoscope cleaning, disinfecting and sterilizing device 101, the first treatment unit 112 for cleaning or cleaning and disinfecting the endoscope 105 and the second treatment unit 120 for sterilizing the endoscope 105 are integrated and incorporated in the device main body 102, and therefore cost of the whole device can be reduced. Furthermore, all the processes of the cleaning and disinfecting and sterilizing treatment of the endoscope 105 are controlled by the main body control circuit 158 of the endoscope cleaning, disinfecting and sterilizing device, 101, and all the cleaning and disinfecting and sterilizing can be automatically performed. Therefore, the whole device can be miniaturized as compared with a case where the treatment unit for cleaning or cleaning and disinfecting the endoscope 105 is independently separated from the treatment unit for sterilizing the endoscope 105. Therefore, the device 1 which easily performs the respective cleaning and disinfecting and sterilizing operations can be installed even in a general consultation room where the diagnosis is conducted with the endoscope 105.

Moreover, when the endoscope 105 is conveyed between the first treatment section 110A and second treatment section 110B by the tray conveying means 125 in the device main body 102, a time required for the respective cleaning and disinfecting and sterilizing operations of the endoscope 105 or the like performed between cases of the endoscopic diagnosis can be reduced, and efficiency of the endoscopic diagnosis can be enhanced. Furthermore, since the endoscope 105 does not have to be set up every cleaning and disinfecting and sterilizing operation process of the endoscope 105 or the like as in the related art, the number of user's reprocesses can be largely reduced.

Furthermore, since the tray 106 for the cleaning and disinfecting and sterilizing is used in common in the device main body 102, and all the cleaning, disinfecting, and sterilizing treatments can be automatically performed, the whole device can be miniaturized, and product cost can be largely reduced. Furthermore, since the tray 106 is detachably attached to the device main body 102, it is possible to hold the state of the endoscope 105 without changing the state in and after all the cleaning, disinfecting, and sterilizing processes.

Moreover, since the tray 106 is detachably attached to the device main body 102, a plurality of endoscopes and the like can be treated in order in the same device 101. Therefore, operation ratio of the device 101 can be enhanced, and a running cost of the whole reprocess can be largely reduced. Furthermore, since the tray 106 for the cleaning and disinfecting and sterilizing is used in common, and all the cleaning, disinfecting, and sterilizing operation processes of the endoscope 105 and the like can be automatically performed, a reprocess time is reduced, a reprocess between the cases is possible, and the endoscopic inspection can be conducted with good efficiency.

It is to be noted that the cleaning section for use during the cleaning of the endoscope 105 is generally water, or water mixed with detergent for use, but instead of a method of storage in the cleaning tank 113, a water supply pipe line may also be connected directly to a city water pipe line or the like in the method.

Moreover, the cleaning and disinfecting processes are performed in the first treatment section 110A, but disinfecting means and disinfecting process are omitted, and only the cleaning and sterilizing processes may be performed in the device.

Furthermore, FIGS. 15 and 16 show a further example. In the example, the constitution of each valve 131 for opening and closing the pipe line of the tray 106 in the endoscope cleaning, disinfecting and sterilizing device 101 (see FIG. 7 to FIGS. 14A to 14C) is modified as follows.

That is, the constitution has been described in which the electric circuit applying the radio frequency identification (RFID) using the radio waves is disposed as the driving mechanism of the valve 131 for opening and closing the pipe line between the main body side transmission and reception units 117b, 124b and the tray side transmission and reception unit 144. Instead of this constitution, a mechanical driving mechanism 171 of the valve 131 for opening and closing the pipe line is disposed.

In the driving mechanism 171 of the valve 131 for opening and closing the pipe line, a cam member 172 is fixed to the shaft 140 of the plunger 139 for opening and closing the valve body 136 in the valve 131 for opening and closing the pipe line. A semicircular cam surface 173 is formed in this cam member 172.

A pin insertion port 174 is formed in the side surface of the pipe line constituting member 135 of the valve 131 for opening and closing the pipe line. As shown in FIG. 16, the pin insertion port 174 is disposed in a portion which is detached from or faces the cam surface 173 of the cam member 172 in a state in which the valve body 136 of the valve 131 for opening and closing the pipe line is allowed to abut on the valve seat portion of the peripheral edge of the communicating port 137 and is urged in a direction for closing the communicating port 137 by the spring force of the coil spring 143.

Furthermore, a cam driving pin 175 is protruded from the wall surface in the vicinity of the tray side pipe line connecting portion 117a in the tray connecting portion 111A of the first treatment section 110A of the endoscope cleaning, disinfecting and sterilizing device 101 (tray-side pipe line connecting portion 124a in the tray connecting portion 111B of the second treatment section 110B). The cam driving pin 175 is detachably inserted into the pin insertion port 174 of the pipe line constituting member 135 of the valve 131 for opening and closing the pipe line. Moreover, when the cam driving pin 175 is inserted into the pin insertion port 174, the tip portion of the cam driving pin 175 abuts on the cam surface 173 of the cam member 172. At this time, the shaft 140 of the plunger 139 moves in the upward direction in FIG. 15 by a cam function of the cam surface 173 of the cam member 172 to switch the valve 131 for opening and closing the pipe line into the released state.

Next, the function will be described. After the end of the diagnosis by the endoscope 105, the endoscope 105 is attached to the tray 106, and the tube for cleaning and disinfecting 159 is connected to the endoscope 105. Thereafter, the tray 106 is set in the first treatment section 110A to clean or clean and disinfect the endoscope 105 in a state in which the lid 108 of the tray 106 is closed.

At this time, the supply pipe line 116 of the tray side pipe line connecting portion 117a in the tray connecting portion 111A of the first treatment section 110A is connected to the pipe line of the pipe line constituting member 135 of two valves 131a, 131b for opening and closing the pipe lines on the left-side surface of the tray 106. Furthermore, the cam driving pin 175 in the vicinity of the tray side pipe line connecting portion 117a in the tray connecting portion 111A of the first treatment section 110A of the endoscope cleaning, disinfecting and sterilizing device 101 is inserted into the pin insertion port 174. Here, the tip portion of the cam driving pin 175 inserted in the pin insertion port 174 abuts on the cam surface 173 of the cam member 172, accordingly the shaft 140 of the plunger 139 moves in the upward direction in FIG. 15 by the cam function of the cam surface 173 of the cam member 172, and the valve 131 for opening and closing the pipe line is switched into the released state. Accordingly, the pipe line for the cleaning and disinfecting is secured. Moreover, the pipe line of the cleaning and disinfecting tube 159 is also secured. Furthermore, the valve 131 for opening and closing the pipe line is similarly switched in the released state also in the portion of the solution discharge valve 133 to secure the solution discharge pipe line. In this state, the cleaning and disinfecting processes are performed in the same manner.

Moreover, after the cleaning and disinfecting processes are completed, the tray 106 is removed from the first treatment section 110A by the tray conveying means 125. At this time, with the operation for removing the tray 106 from the first treatment section 110A, the cam driving pin 175 is pulled from the pin insertion port 174. Therefore, in this state, as shown in FIG. 16, the valve body 136 of the valve 131 for opening and closing the pipe line is allowed to abut on the valve seat portion of the peripheral edge of the communicating port 137, and the communicating port 137 is closed by the spring force of the coil spring 143. At this time, the pipe line of the cleaning and disinfecting tube 159 is also closed. Furthermore, since the solution discharge valve 133 is also closed, and the tray 106 is sealed and closed in a state in which the endoscope 105 is stored, the endoscope 105 is prevented from being contaminated from the outside of the tray 106.

Thereafter, the tray 106 is conveyed to the second treatment section 110B by the tray conveying means 125. Moreover, when the tray 106 is attached to the second treatment section 110B in the same manner as before, the high-pressure steam supply pipe line 123 of the tray-side pipe line connecting portion 124a in the tray connecting portion 111B of the second treatment section 110B is connected to the pipe line of the pipe line constituting member 135 of two valves 131c, 131d for opening and closing the pipe lines on the right-side surface of the tray 106. At this time, the cam driving pin 175 in the vicinity of the tray-side pipe line connecting portion 124a in the tray connecting portion 111B of the second treatment section 110B is inserted into the pin insertion port 174. Accordingly, the shaft 140 of the plunger 139 moves in the upward direction in FIG. 15 by the cam function of the cam surface 173 of the cam member 172 to switch the valve 131 for opening and closing the pipe line into the released state, the pipe line for supplying the high-pressure steam is secured, and the preparation for the sterilizing process is completed. Thereafter, the processes similar to those above are performed, and all the processes are completed.

Then, the mechanical driving mechanism 171 of the valve 131 for opening and closing the pipe line is disposed, the electric driving circuit is not used as the driving mechanism of the valve 131 for opening and closing the pipe line, and therefore heat resistance is also enhanced. Furthermore, the electric circuit in the vicinity of the second treatment section 110B as the sterilization chamber which easily reaches at the high temperature can be reduced, therefore the sterilization treatment at the high temperature (e.g., about 140°C) is further possible, and the time reduction, sterilization capability enhancement and the like of the sterilization treatment can be achieved with the raising of the sterilization temperature.

Furthermore, since the structure of the valve 131 for opening and closing the pipe line can be simplified, further miniaturization is possible by reduction of constituting components, and further the reduction of the cost of the device 101 is possible.

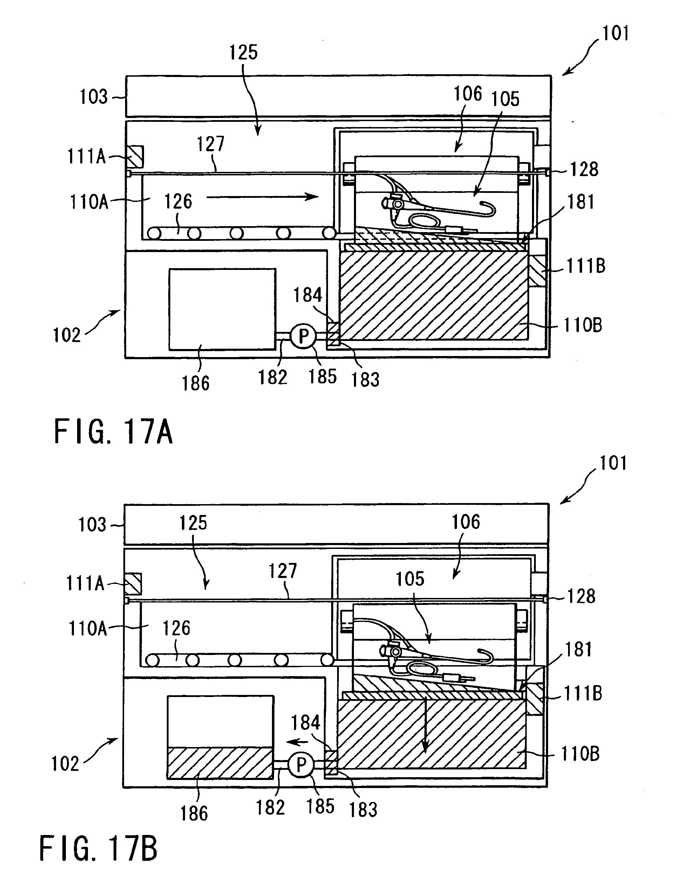

Moreover, FIGS. 17A to 17C show a further example, not forming part of the claimed invention. The constitution of means for lifting up and down the tray 106 in the vertical moving path 109b of the tray moving path 109 in the endoscope cleaning, disinfecting and sterilizing device 101 (FIG. 7 to FIGS. 14A to 14C) is modified as follows.

That is, as shown in FIG. 17A, a float 181 is disposed to float the tray 106 using buoyancy of the liquid in the second treatment section 110B. Furthermore, a connection portion 183 connected to one end of a liquid pipe line 182 is disposed in the bottom part of the second treatment section 110B. An electromagnetic valve 184 for controlling the supply and discharge of the liquid into the second treatment section 110B is disposed in the connection portion 183.

Moreover, the other end of the liquid pipe line 182 is connected to a fluid tank 186 for storing the liquid via a fluid pump 185. Furthermore, when the fluid pump 185 is driven, the liquid is supplied into the second treatment section 110B from the fluid tank 186 and the liquid is discharged from the second treatment section 110B. Furthermore, the supply and discharge of the liquid into the second treatment section 110B is controlled by the electromagnetic valve 184. Accordingly, increase or decrease of the liquid to be supplied into the second treatment section 110B is adjusted, and the height of the float 181 in the second treatment section 110B is adjusted.

Moreover, the tray 106 conveyed on the second treatment section 110B side from the first treatment section 110A is laid on the float 181 in the second treatment section 110B. Moreover, while the tray 106 is floated on the liquid by the buoyancy of the float 181, the supply and discharge of the liquid in the second treatment section 110B are controlled by the electromagnetic valve 184, and accordingly the tray 106 is lifted up and down in the vertical moving path 109b of the tray moving path 109.

Next, the function will be described. When the cleaning and disinfecting process is completed in the same manner as before in the first treatment section 110A, the electromagnetic valve 184 is released, and the fluid pump 185 operates. Accordingly, the liquid stored in the fluid tank 186 is supplied into the second treatment section 110B to raise the float 181. At this time, the float 181 is raised to a position substantially horizontal with respect to the belt conveyor 126.

Thereafter, the tray conveying means 125 conveys the tray 106 above the second treatment section 110B. Moreover, when the rail control unit 128 releases the tray 106 in the terminal end position of the rail 127 for conveying the tray, as shown in FIG. 17A, the tray 106 is laid on the float 181.

Next, when the fluid pump 185 is driven in a direction for recovering the liquid in the second treatment section 110B, as shown in FIG. 17B, a liquid level in the second treatment section 110B lowers, and the tray 106 is conveyed below the second treatment section 110B.

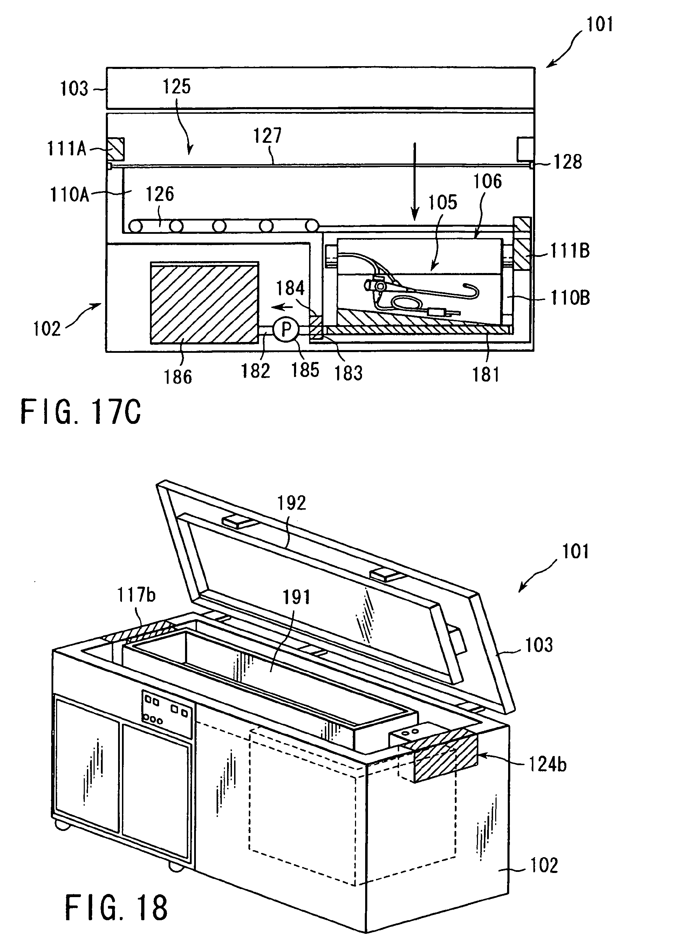

Moreover, as shown in FIG. 17C, the tray 106 is set in the predetermined set position inside the second treatment section 110B, and the sterilizing process is executed in the same manner as before.

Moreover, after the end of the sterilizing process, the liquid is supplied into the second treatment section 110B by the fluid pump 185, the liquid level in the second treatment section 110B is accordingly raised, the tray 106 on the float 181 is lifted up, and an operation of returning the tray 106 to the initial position is performed.

Then, the above-described constitution produces the following effect. That is, when the liquid surface in the second treatment section 110B is adjusted using a fluid control technique, the tray 106 in the second treatment section 110B is accordingly lifted up and down. Therefore, as compared with the use of complicated mechanical tray lifting-up and down means, the device 101 can be miniaturized, the cost can be reduced, and quality can be stabilized.

Moreover, the fluid tank 186 is usable in common for the cleaning section, water for generating the high-pressure steam and the like. Therefore, by the reduction of the constituting components, the device 101 can further be miniaturized, and the cost reduction can be effectively realized.

Furthermore, FIG. 18 shows a further example, not forming part of the claimed invention. The constitutions of the tray 106 and device main body 102 in the endoscope cleaning, disinfecting and sterilizing device 101 (see FIG. 7 to FIGS. 14A to 14C) are modified as follows.

That is, a tray 191 which also serves as the sterilization chamber is disposed above the substantially box-shaped device main body 102. Furthermore, the lid body 103 of the device main body 102 is integrated with a tray lid 192 of the tray 191.

Moreover, the second main body side transmission and reception unit 124b is disposed in the vicinity of the tray 191. It is to be noted that the tray conveying means 125 is omitted, and the other structure and constitution of the valve 131 for opening and closing the pipe line of the tray main body, various transmission and reception units, cleaning unit, and sterilizing unit are similar to those mentioned before.

Furthermore, the tray main body of the tray 191 is detachably connected to the device main body 102. Moreover, a preliminary tray 191 is attached to the device main body 102, and it is possible to further enhance the efficiency in all the processes.

Then, constituted as described above, the tray 191 which also serves as the sterilization chamber is disposed in the upper part of the device main body 102, and the lid body 103 of the device main body 102 is integrated with the tray lid 192 of the tray 191. Therefore, the whole endoscope cleaning, disinfecting and sterilizing device 101 can further be miniaturized, and the cost reduction can be achieved.

Furthermore, after the completion of the sterilization, the endoscope 105 can be easily removed from the tray 191, and the number of user's disinfecting and sterilizing treatment processes can further be reduced.

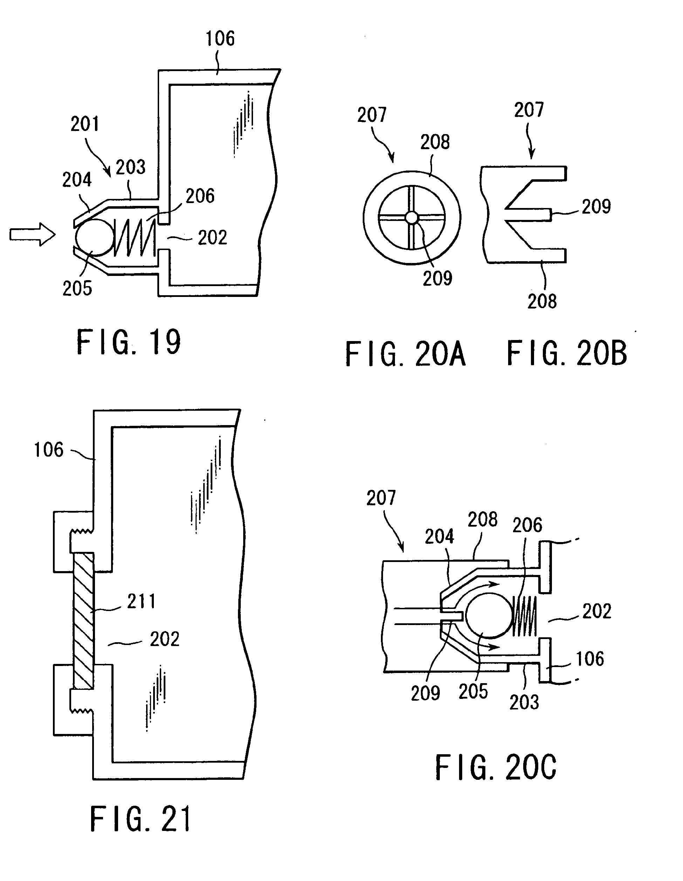

Moreover, FIGS. 19 and 20A to 20C show a further example, not forming part of the claimed invention. The constitution of the valve 131 for opening and closing the pipe line of the tray 106 in the endoscope cleaning, disinfecting and sterilizing device 101 (see FIG. 7 to FIGS. 14A to 14C) is modified as follows.

That is, a check valve 201 which opens by application of the pressure from the outside is disposed in the tray 106. The check valve 201 is provided with a cylindrical casing 203. The casing 203 is protruded outwards from the periphery of a liquid inflow hole 202 formed in the side wall of the tray 106. A tapered valve receiving portion 204 is formed in the tip portion of the casing 203.

Furthermore, a ball-shaped valve body 205 and a coil spring 206 are disposed in the casing 203. The valve body 205 opens and closes a tip opening 204a of the valve receiving portion 204. The coil spring 206 urges the valve body 205 can be held the tip opening 204a of the valve receiving portion 204 is closed.

Moreover, tray connecting connectors 207 shown in FIGS. 20A, 20B are disposed in the first and second treatment sections 110A and 110B of the device main body 102. A connector main body 208 detachably connected to the casing 203 of the check valve 201 is disposed in the connector 207. A bar-shaped operation rod 209 is protruded from an axial center portion of the connector main body 208. This operation rod 209 is inserted into the tip opening 204a of the valve receiving portion 204.

Furthermore, when the tray 106 is set in the first treatment section 110A or the second treatment section 110B of the device main body 102, as shown in FIG. 20C, the check valve 201 of the tray 106 is inserted into and connected to the connector 207 of the first treatment section 110A or the second treatment section 110B. At this time, the operation rod 209 of the connector 207 is inserted into the tip opening 204a of the valve receiving portion 204 in the check valve 201. Accordingly, the valve body 205 of the check valve 201 is pushed in a direction far from the tip opening 204a of the valve receiving portion 204 against the spring force of the coil spring 206. Therefore, since the tip opening 204a of the valve receiving portion 204 is released, the check valve 201 is switched and operated in the open state to secure the flow path of the tray 106.

Then, the above-described constitution produces the following effect. That is, the check valve 201 which opens by the application of the pressure from the outside is disposed in the tray 106. Therefore, even without the electric control means such as the first main body side transmission and reception unit 117b and second main body side transmission and reception unit 124b, liquid or gas or steam for the cleaning or disinfecting or sterilizing can be easily supplied into the tray 106, and the constitution can be simplified. Furthermore, in a case other than the case where the tray 106 is set in the first treatment section 110A or the second treatment section 110B of the device main body 102, the valve body 205 can be held by the spring force of the coil spring 206 in the check valve 201 in a state in which the tip opening 204a of the valve receiving portion 204 is closed. As a result, foreign matters can be prevented from being mixed into the tray 106.

It is to be noted that the check valve 201 of the tray 106 may also be structured to open when the connector 207 of the device main body 102 is inserted and to close when the connector is pulled out. The valve may also be structured to open by a pressing force from the pressurized and fed liquid or gas or steam and to close when any pressure is not applied.

Moreover, FIG. 21 shows a modification of this example. In the present modification, a membrane filter film 211 in which micro holes having a hole diameter (e.g., about 0.2 micron) incapable of passing bacteria are formed is attached so as to cover the liquid inflow hole 202 of the tray 106. Moreover, when the tray 106 is set in the first treatment section 110A or the second treatment section 110B of the device main body 102, only the pressurized and fed liquid or gas or steam passes through the membrane filter film 211 to flow in the liquid inflow hole 202 of the tray 106.

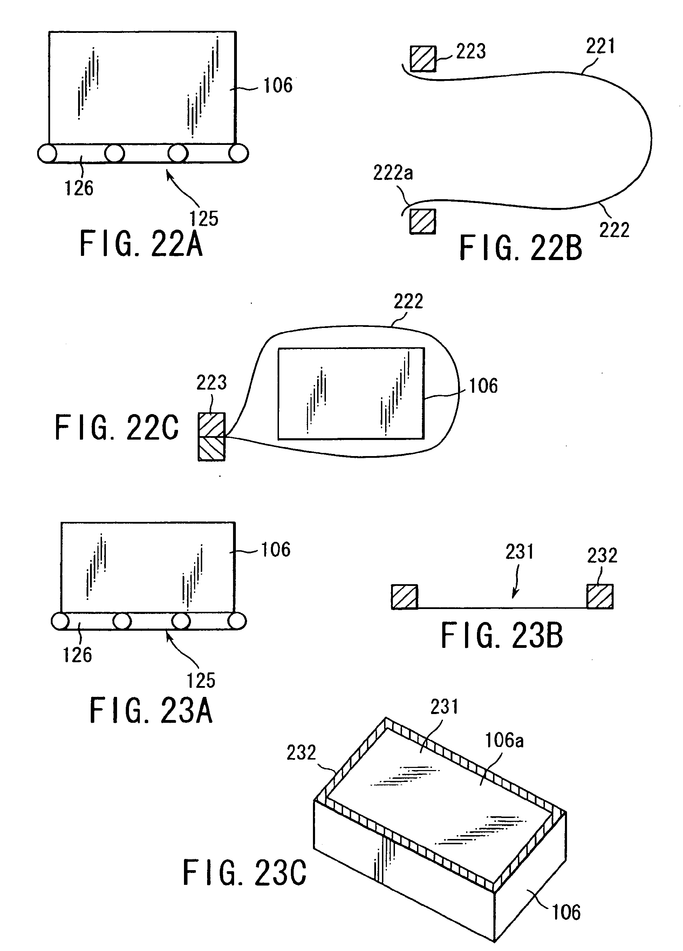

Moreover, FIGS. 22A to 22C show an example not forming part of the claimed invention. The constitution of the endoscope cleaning, disinfecting and sterilizing device 101 (see FIG. 7 to FIGS. 14A to 14C) is modified as follows.

That is, a bag 222 is disposed, and the whole tray 106 is covered with a film 221 (material or the like of a commercially available sterilized pack) capable of passing gas or steam but incapable of passing the foreign matters such as bacteria and dust. Moreover, during the conveying of the tray 106 between the first and second treatment sections 110A and 110B of the device main body 102, or after conveying the tray 106 into the second treatment section 110B, the whole tray 106 is constituted to be covered with the bag 222. Here, a thermally welded portion 223 is disposed in the peripheral edge of an opening 222a of the bag 222.

Moreover, the bag 222 is on standby in the terminal end position of a conveying track of the tray 106 conveyed by the belt conveyor 126 from the first treatment section 110A. Moreover, after the tray 106 conveyed by the belt conveyor 126 is contained in the bag 222, the opening 222a of the bag 222 is closed and the thermally welded portion 223 is thermally welded to seal the bag.

Then, the whole tray 106 is not sealed by the film 221 during the cleaning and disinfecting processes. Therefore, the endoscope 105 in the tray 106 may be subjected to spray cleaning of the cleaning section or immersion disinfection in the antiseptic solution in an open environment. Furthermore, during the conveying of the tray 106 between the first and second treatment sections 110A and 110B of the device main body 102, or after conveying the tray 106 into the second treatment section 110B, the tray 106 is contained in the bag 222, the opening 222a of the bag 222 is closed in this state, and the thermally welded portion 223 is thermally welded to seal the bag. Therefore, the whole tray 106 can be covered and sealed with the film 221 capable of passing the gas or steam but incapable of passing the foreign matters such as bacteria and dust before the injection of the high-pressure steam, and it is possible to maintain cleanness after the sterilization treatment.

Moreover, FIGS. 23A to 23C show a modification of this example. In the present modification, only an upper surface opening 106a of the tray 106 is automatically covered with a sheet film 231. Moreover, a thermally welded portion 232 disposed in the peripheral edge of the sheet film 231 is thermally welded to the peripheral edge of the upper surface opening 106a of the tray 106 to achieve a sealed constitution.

## Claims

1. An endoscope cleaning and sterilizing device in which an endoscope is cleaned and subjected to a high temperature and pressure steam sterilization, the device comprising:
a container (34) in which the endoscope (2) is stored and subjected to the high temperature and pressure steam sterilization;
a cleaning section (40) to store the container (34) in a state in which the
endoscope is stored and to clean the endoscope in the container; and
a high temperature and pressure steam sterilization section (50) to store the container (34) in a state in which the endoscope remains to be stored and to subject the endoscope (2) in the container to the high temperature and
pressure steam sterilization,
**characterized in that** said device comprises a posture adjustment portion (61d) to store the endoscope (2) in a state in which at least one of the openings (25) of a pipeline constituting body is directed and disposed in a direction other than a gravity direction.

2. The endoscope cleaning and sterilizing device according to claim 1 wherein said posture adjustment portion (61d) is formed in said container (34, 35).

3. The endoscope cleaning and sterilizing device according to claim 1 or 2, wherein the device comprise a hanger device from which the endoscope is to be hung, and
the hanger device comprises a hanger base portion, and a hanger head detachably attached to the hanger base portion and capable of being subjected to the high temperature and pressure steam sterilization.

4. The endoscope cleaning and sterilizing device according to claim 1, wherein the hanger head is detachably attached to the hanger base portion in a combined state with the endoscope.

## Patentansprüche

1. Eine Reinigungs- und Sterilisationsvorrichtung für Endoskope, in welcher ein Endoskop gereinigt und einer Hochtemperatur- und Dampfdrucksterilisation unterworfen wird, wobei die Vorrichtung aufweist:
einen Behälter (34), in welchem das Endoskop (2) aufgenommen und der Hochtemperatur- und Dampfdrucksterilisation unterworfen wird;
einen Reinigungsabschnitt (40) zur Aufnahme des Behälters (34) in einem Zustand, in welchem das Endoskop aufgenommen ist und zur Reinigung des Endoskops in dem Behälter; und
einen Hochtemperatur- und Dampfdrucksterilisationsabschnitt (50) zur Aufnahme des Behälters (34) in einem Zustand, in welchem das Endoskop aufgenommen verbleibt und um das Endoskop (2) in dem Behälter der Hochtemperatur- und Dampfdrucksterilisation zu unterwerfen,
**dadurch gekennzeichnet, dass**
die Vorrichtung einen Ausrichtungseinstellabschnitt (61d) aufweist zur Aufnahme des Endoskops (2) in einem Zustand, in welchem wenigstens eine der Öffnungen (25) eines eine Leitung bildenden Körpers in eine von der Schwerkraftrichtung abweichende Richtung ausgerichtet und angeordnet ist.

2. Die Reinigungs- und Sterilisationsvorrichtung für Endoskope nach Anspruch 1, wobei der Ausrichtungseinstellabschnitt (61d) in dem Behälter (34, 35) ausgebildet ist.

3. Die Reinigungs- und Sterilisationsvorrichtung für Endoskope nach Anspruch 1 oder 2,
wobei die Vorrichtung eine Aufhängevorrichtung aufweist, an der das Endoskop aufhängbar ist, und
wobei die Aufhängevorrichtung einen Aufhängebasisabschnitt und einen Aufhängekopf aufweist, der lösbar an dem Aufhängebasisabschnitt angebracht ist und der Hochtemperatur- und Dampfdrucksterilisation unterwerfbar ist.

4. Die Reinigungs- und Sterilisationsvorrichtung für Endoskope nach Anspruch 1, wobei der Aufhängekopf an dem Aufhängebasisabschnitt in einem mit dem Endoskop kombinierten Zustand lösbar angebracht ist.

## Revendications

1. Dispositif de nettoyage et de stérilisation pour endoscope, dans lequel un endoscope est nettoyé et soumis à une stérilisation à la vapeur sous haute température et haute pression, le dispositif comprenant :
un récipient (34), dans lequel l'endoscope (2) est stocké et soumis à la stérilisation à la vapeur sous haute température et haute pression ;
une section de nettoyage (40), pour stocker le récipient (34) dans un état dans lequel l'endoscope est stocké, et pour nettoyer l'endoscope dans le récipient ; et
une section de stérilisation à la vapeur sous haute température et haute pression (50), pour stocker le récipient (34) dans un état dans lequel l'endoscope reste à stocker et pour soumettre l'endoscope (2) dans le récipient à la stérilisation à la vapeur sous haute température et haute pression ;
**caractérisé en ce que** ledit dispositif comprend une partie d'ajustement de posture (61d), pour stocker l'endoscope (2) dans un état dans lequel au moins l'une des ouvertures (25) d'un corps constitué d'une tuyauterie est orientée et disposée dans une direction autre que la direction de la gravité.

2. Dispositif de nettoyage et de stérilisation pour endoscope selon la revendication 1, dans lequel ladite partie d'ajustement de posture (61d) est formée dans ledit récipient (34, 35).

3. Dispositif de nettoyage et de stérilisation pour endoscope selon la revendication 1 ou 2, dans lequel le dispositif comprend un dispositif de suspension, d'où l'endoscope doit être suspendu, et
le dispositif de suspension comprend une partie de base de suspension, et une tête de suspension attachée de façon détachable à la partie de base de suspension et capable d'être soumise à la stérilisation à la vapeur sous haute température et haute pression.

4. Dispositif de nettoyage et de stérilisation pour endoscope selon la revendication 1, **caractérisé en ce que** la tête de suspension est attachée de façon détachable à la partie de base de suspension, dans un état combiné à l'endoscope.
